# EUROPÄISCHE PATENTSCHRIFT

(11) **EP 1 951 308 B1**
(45) Veröffentlichungstag und Bekanntmachung des Hinweises auf die Patenterteilung: **20.02.2013**
(21) Anmeldenummer: 06818110.6
(22) Anmeldetag: 27.11.2006
(51) Int. Cl.: A61K 45/06, A61K 31/685, A61K 31/14, A61K 31/7068, A61K 31/7072, A61P 25/00, A61P 25/16, A61P 25/28

(54) **KOMBINATIONSPRÄPARATE ENTHALTEND PHYSIOLOGISCHE ZELLMEMBRAN-BESTANDTEILE, EINSCHLIESSLICH PHOSPHATIDYLSERIN, CHOLIN UND EIN PYRIMIDINNUKLEOS/TID**
COMBINATION PREPARATIONS CONTAINING PHYSIOLOGICAL CELL MEMBRANE CONSTITUENTS INCLUDING PHOSPHATIDYLSERINE, CHOLINE AND A PYRIMIDINE NUCLEOSIDE/NUCLEOTIDE
ASSOCIATIONS MEDICAMENTEUSES CONTENANT DES ELEMENTS PHYSIOLOGIQUES DE LA MEMBRANE CELLULAIRE, NOTAMMENT DE LA PHOSPHATIDYLSERINE, DE LA CHOLINE ET UN NUCLEOSIDE/NUCLEOTIDE PYRIMIDIQUE

(30) Priorität: 25.11.2005 DE 102005056558; 06.12.2005 US 742534 P
(43) Veröffentlichungstag der Anmeldung: 06.08.2008
(73) Patentinhaber: Susilo, Gisela, 50999 Köln (DE)
(72) Erfinder: Susilo, Gisela, 50999 Köln (DE)
(74) Vertreter: Arth, Hans-Lothar
(86) Internationale Anmeldenummer: PCT/DE2006/002091
(87) Internationale Veröffentlichungsnummer: WO 2007/059762

(56) Entgegenhaltungen:
- WO-A-00/06174
- WO-A-01/68104
- WO-A-2005/023271
- WO-A-2005/037262
- WO-A-2006/127620
- US-A1- 2005 031 651
- US-A1- 2005 176 676
- MURALIKRISHNA ADIBHATLA ET AL: "CDP-choline liposomes provide significant reduction in infarction over free CDP-choline in stroke" BRAIN RESEARCH, ELSEVIER, AMSTERDAM, NL, Bd. 1058, Nr. 1-2, 5. Oktober 2005 (2005-10-05), Seiten 193-197, XP005107537 ISSN: 0006-8993

## Beschreibung

Die Erfindung betrifft Kombinationspräparate umfassend Phosphatidylserin, mindestens ein Pyrimidinnukleosid oder mindestens ein Pyrimidinnukleotid und Cholin bzw. aus den physiologisch verträglichen Salzen dieser Verbindungen sowie Zusammensetzungen enthaltend diese Kombination aus den vorgenannten Wirkstoffen. Die Erfindung betrifft ferner die Verwendung einer Kombination umfassend Phosphatidylserin und Cytidindiphosphocholin oder Phosphatidylserin und Cholin und mindestens ein Pyrimidinnukleosid oder mindestens ein Pyrimidinnukleotid oder einer Zusammensetzung enthaltend besagte Kombination zusammen mit mindestens einem physiologisch verträalichen Träger, Zusatzstoff, Hilfsstoff und/oder Lösungsmittel zur Herstellung einer pharmazeutischen Zusammensetzung zur Prophylaxe und/oder Behandlung von neurodegenerativen und entzündlichen Erkrankungen.

Lecithine und Kephaline unterscheiden sich in ihrem Aufbau durch unterschiedliche organische basische Reste an der Phosphorsäuregruppe. Wird die Aminosäure Serin an die Phosphatgruppe gebunden, so gelangt man zum Serinkephalin, welches auch als Phosphatidylserin bezeichnet wird und folgende Struktur besitzt:

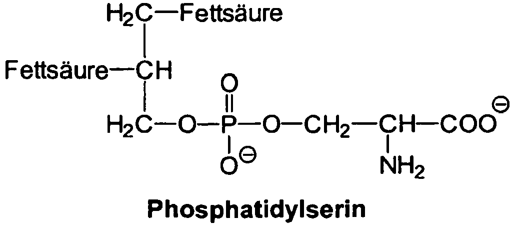

Das Phosphatidylserin enthält zwei beliebige Fettsäurereste, welche mit dem Glycerinrest verestert sind. Handelt es sich beispielsweise um zwei Palmitoylfettsäurereste, so erhält man Dipalmitoylphosphatidylserin folgender Struktur 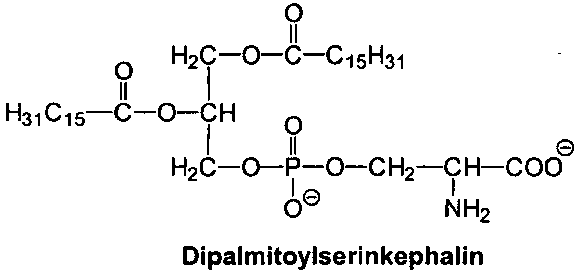 Phosphatidylserin wird insbesondere zur Behandlung von Alzheimer, Depressionen, Gedächtnisschwächen, bei allgemein herabgesetzter mentaler und physischer Leistungsfähigkeit und bei diversen Altersbeschwerden eingesetzt.

Cholin wird insbesondere als Neurotransmitter-Vorstufe verwendet und hat folgende Struktur

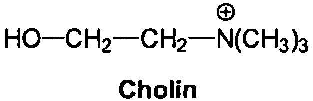

Als der Vorstufe von Cholin und Cytidin kann auch Cytidindiphosphocholin (CDP-Cholin) eingesetzt werden.

Cytidin und/oder Uridin und/oder deren Nukleotide [Cytidinmonophoshat (CMP), Cytidindiphosphat (CDP), Cytidintriphosphat (CTP), Uridinmonophosphat (UMP), Uridindiphosphat (UDP), Uridintriphosphat (UTP)] werden zur Unterstützenden Behandlung bei allgemeinen neurodegenerativen Erkrankungen eingesetzt. Cytidin bzw. Uridin besitzen folgende Struktur:

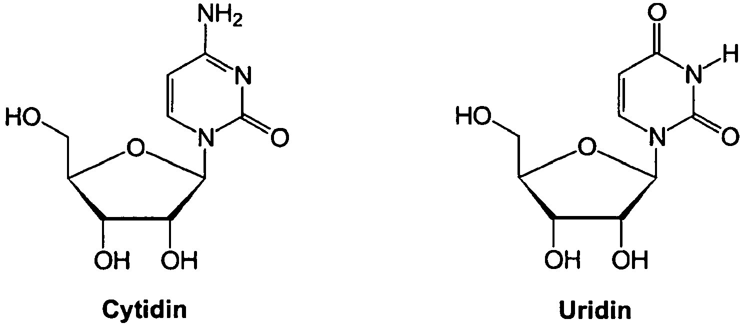

US 2005/176676 A1 offenbart eine Behandlungsmethode mit Uridin für kognitive Erkrankungen. Eine zweite Substanz kann dabei synergistisch eingesetzt werden. Diese zweite Substanz kann Cholin, ein Cholin-Salz oder eine Vorstufe von Cholin, wie z.B. Citicolin oder CDP-Cholin. Es wird außerdem offenbart, dass eine Behandlung mit Uridin sowohl die Level an Phosphatidylserin als auch an Cytidin erhöht. Eine Kombination von Uridin mit Cholin und Phosphatidylserin oder von CDP-Cholin und Phosphatidylserin wird in der US 2005/176676 A1 nicht erwähnt.

Aufgabe der vorliegenden Erfindung ist es, Wirkstoffkombinationen und Zusammensetzungen zur Prophylaxe und Behandlung von entzündlichen und neurodegenerativen Erkrankungen bereitzustellen.

Diese Aufgabe wird durch die technische Lehre der unabhängigen Patentansprüche gelöst. Vorteilhafte Ausgestaltungen der Erfindung sind in den abhängigen Patentansprüchen, der Beschreibung sowie den Beispielen angegeben.

Überraschenderweise wurde gefunden, dass die Kombination aus Phosphatidylserin und Cytidindiphosphocholin bzw. die Kombination aus Phosphatidylserin, einem Pyrimidinnukleosid oder Pyrimidinnukleotid und Cholin den Einzelsubstanzen als auch der Kombination aus Cholin und einem Pyrimidinnukleosid wie z.B. Cytidin bei der Stimulation der Hirnaktivität überlegen ist.

Somit betrifft die vorliegende Erfindung die Kombination aus Phosphatidylserin, Cholin und Cytidin/Uridin und deren Nukleotiden Cytidinmonophosphat, Cytidindiphosphat, Cytidintriphosphat, Uridinmonophosphat, Uridindiphosphat, Uridintriphosphat. Cytidin wird im Organismus zu Uridin umgewandelt bzw. umgekehrt. Ferner ist auch bekannt, dass unter physiologischen Bedingungen die Cytidinnukleotide in die entsprechenden Uridinnukleotide umgewandelt werden können und umgekehrt.

Somit ist die vorliegende Erfindung auf synergistische pharmazeutische Kombinationen aus a) und b) gerichtet, sowie die Verwendung der Kombinationen aus a) oder b) oder c) zur Vorbeugung und Behandlung entzündlicher als auch gastrointestinaler Erkrankungen und insbesondere neurodegenerativer Erkrankungen sowie Hirnleistungsstörungen und Hirnleistungsstörungen nach Schlaganfall.
a) Phosphatidylserin und Cholin und Uridin oder ein Derivat von Uridin oder
b) Phosphatidylserin und Cholin und Cytidin oder ein Derivat von Cytidin oder
c) Phosphatidylserin und Cytidindiphosphocholin

Die Dreierkombinationen gemäß a) und b) sowie die Zweierkombination gemäß c) weisen synergistische Effekte auf, welche die Einzelverbindungen oder auch Zweierkombinationen ohne Cytidindiphosphocholin nicht besitzen.

Die folgende Tabelle stellt die getesteten Kombinationen und Verbindungen gemäß den Studienbeispielen 1 - 5 zusammen (+ bedeutet signifikante Reduzierung der delta Aktivität und Steigerung der Hirnleistung und Hirnaktivität und neuro-regenerierende Wirkung; - bedeutet keine oder keine signifikante Reduzierung der delta Aktivität und keine bzw. keine signifikante Steigerung der Hirnleistung und Hirnaktivität und keine neuro-regenerierende Wirkung):

| **Wirkstoff(e)** | **neuro-regenerierende Wirkung** |
|---|---|
| Phosphatidylserin | - |
| Cytidin + Cholin | - |
| Phosphatidylserin + Cytidin + Cholin (erfindungsgemäβ) | + |
| CDP-Cholin | - |
| Phosphatidylserin | - |
| Phosphatidylserin + CDP-Cholin (erfindungsgemäß) | + |
| CMP + Cholin + Phosphatidylserin (erfindung | + |
| UMP + Cholin + Phosphatidylserin (erfindungsgemäß) | + / - |
| dUTP + Cholin + Phosphatidylserin (erfindungsgemäß) | + |
| dUMP + Phosphatidylserin | - |
| Cholin + Phosphatidylserin | - |

Bevorzugte neurodegenerative Erkrankungen sind Alzheimer Erkrankung, Parkinson'sche Krankheit, Demenz, ADHS, Huntington'sche Krankheit, Hirnleistungsstörungen und Hirnleistungsstörungen nach Schlaganfall.

Als Derivat von Uridin werden Deoxyuridin, Uridinmonophosphat, Deoxyuridinmonophosphat, Uridindiphosphat, Deoxyuridindiphosphat, Uridintriphosphat sowie Deoxyuridintriphosphat und deren Salze bezeichnet.

Als Derivat von Cytidin werden Deoxycytidin, Cytidinmonophoshat, Deoxycytidinmonophoshat, Cytidindiphosphat, Deoxycytidindiphosphat, Cytidintriphosphat sowie Deoxycytidintriphosphat und deren Salze bezeichnet.

Um Cholin und Cytidin im Körper zu substituieren ist es auch möglich Cytidindiphosphocholin (CDP-Cholin) als Vorstufe zu applizieren, da CDP-Cholin bereits vor der Resorption im Magen-Darm-Trakt zu Cholin und Cytidin umgewandelt wird. Somit sind als Vergleich zur vorliegenden Erfindung auch Kombinationen aus Phosphatidylserin und CDP-Cholin möglich.

Das Phosphatidylserin kann beliebige Fettsäurereste tragen und ist kommerziell fast immer als Phospholipid-Mischung erhältlich. Erfindungsgemäß können auch Phosphatidylserin-enthaltende Mischungen eingesetzt werden wie beispielsweise 20% bis 40% PS (1,2-Diacyl-sn-glicero-3-phospho-L-serine), worin der Rest (80% bzw 60%) Phos-choline, Phos-ethanolamine und Phosphatidylenositol sind.

Cholin liegt als Salz, bevorzugt als Chlorid, Citrat, Bitartrat oder Carbonatsalz vor. Der Begriff "Cholin" wie hierin verwendet umfasst natürlich insbesondere die Salze dieser Substanz. Als Anionen werden insbesondere pharmakologisch verträgliche Gegenionen wie vorgenannt verwendet.

Die Pyrimidinnukleoside Cytidin und Uridin liegen meist als Base vor. Die Begriffe "Pyrimidinnukleosid" bzw. "Cytidin" und "Uridin" wie hierin verwendet umfassen selbstverständlich auch die Salze dieser Verbindungen. Als Kationen werden insbesondere pharmakologisch verträgliche Gegenionen wie hierin genannt verwendet.

Die Pyrimidinnukleotide sind als Natriumsalz im Handel zu erhalten. Pyrimidinnukleosid bezeichnet Cytidin, Deoxycytidin, Uridin, Deoxyuridin sowie Derivate der vorgenannten Verbindungen und Mischungen der vorgenannten Verbindungen. Als Derivate der Pyrimidinnukleoside werden beispielsweise die zum Anmeldetag dieser Patentanmeldung bekannten 4'-Alkyl-2'-deoxynucleoside wie 4'-Ethinyl-2'-deoxynucleosid oder 5-Alkylnukleoside oder 5-Arylnukleoside wie 5-Phenylcytosin als auch die Fluorderivate wie 5-Fluoro-2-deoxynukleosid (z.B. 5-Fluoro-2-deoxyuridin) bezeichnet. Als Deoxynukleoside bzw. Deoxynukleotide sind die 2'-Deoxyverbindungen bevorzugt. Somit bezeichnet dCDP vorzugsweise 2'-Deoxycytidine-5'-monophosphate. Die Bezeichnung Pyrimidinnukleotide umfasst Cytidinmonophoshat (CMP), Deoxycytidinmonophoshat (dCMP), Cytidindiphosphat (CDP), Deoxycytidindiphosphat (dCDP), Cytidintriphosphat (CTP), Deoxycytidintriphosphat (dCTP), Uridinmonophosphat (UMP), Deoxyuridinmonophosphat (dUMP), Uridindiphosphat (UDP), Deoxyuridindiphosphat (dUDP), Uridintriphosphat (UTP) und Deoxyuridintriphosphat (dUTP) sowie Derivate der vorgenannten Verbindungen und Mischungen der vorgenannten Verbindungen. Als Derivate von Pyrimidinnukleotiden können die zum Anmeldetag dieser Patentanmeldung bekannten Fluorderivate wie 5-Fluoro-2-deoxynukleotid (z.B. 5-Fluoro-2-deoxyuridinmonophosphat), 3'-Amino-3'-deoxynukleotide wie 3'-Amino-3'-deoxycytidintriphosphat oder die 4'-substituierten-2'-deoxynukleotide bevorzugt die 4'-C-substituierten-2'-deoxynukleotide wie 4'-Ethyl-2'-deoxyuridindiphosphat, 2'-Acylnukleotide oder 3'-Acylnukleotide oder 2',3'-Diacylnukleotide wie 2'-Acetyluridinmonophosphat oder 2',3'-Diacetyluridinetriphosphat genannt werden.

Die Begriffe "Pyrimidinnukleotid" bzw. "CMP", "CDP", "CTP", "dCMP", "dCDP", "dCTP", "UMP", "UDP", "UTP", "dUMP", "dUDP" und "dUTP" wie hierin verwendet umfassen selbstverständlich auch die Salze dieser Verbindungen. Als Kationen werden insbesondere pharmakologisch verträgliche Gegenionen wie hierin genannt verwendet.

Die drei Komponenten der Kombination können somit bevorzugt in Form ihrer Salze eingesetzt werden. Geeignete Beispiele dieser Salze umfassen Säureadditionssalze als auch Alkalimetallsalze. So können Alkalimetallsalze wie das Natriumsalz, das Kaliumsalz, das Lithiumsalz, das Magnesiumsalz, das Calciumsalz und/oder Alkylammoniumsalze eingesetzt werden. Als Säuren, welche ein Säureadditionssalz bilden, können die folgenden genannt werden: Schwefelsäure, Sulfonsäure, Phosphorsäure, Salpetersäure, salpetrige Säure, Perchlorsäure, Bromwasserstoffsäure, Chlorwasserstoffsäure, Ameisensäure, Essigsäure, Propionsäure, Bernsteinsäure, Oxalsäure, Gluconsäure (Glycons., Dextronsäure), Milchsäure, Apfelsäure, Weinsäure, Tartronsäure (Hydroxymalonsäure, Hydroxypropandisäure), Fumarsäure, Zitronensäure, Ascorbinsäure, Maleinsäure, Malonsäure, Hydroxymaleinsäure, Brenztraubensäure, Phenylessigsäure, (o-, m-, p-) Toluylsäure, Benzoesäure, p-Aminobenzoesäure, p-Hydroxybenzoesäure, Salicylsäure, p-Aminosalicylsäure, Methansulfonsäure, Ethansulfonsäure, Hydroxyethansulfonsäure, Ethylensulfonsäure, p-Toluolsulfonsäure, Naphthylsulfonsäure, Naphthylaminsulfonsäure, Sulfanilsäure, Camphersulfonsäure, Chinasäure (Chininsäure), o-Methyl-mandelsäure, Hydrogenbenzolsulfonsäure, Pikrinsäure (2,4,6-Trinitrophenol), Adipinsäure, d-o-Tolylweinsäure. Auch Aminosäuren können für die Salzbildung eingesetzt werden, wie beispielsweise Glycin, Alanin, Valin, Leucin, Isoleucin, Serin, Threonin, Phenylalanin, Tyrosin, Tryptophan, Lysin, Arginin, Histidin, Aspartat, Glutamat, Asparagin, Glutamin, Cystein, Methionin, Ornithin und Prolin, wobei die Aminosäuren Methionin, Tryptophan, Lysin und Arginin bevorzugt sind.

Vorzugsweise werden die Komponenten Phosphatidylserin und Cytidindiphosphocholin bzw. Phosphatidylserin, Cholin und das mindestens eine Nukleosid Cytidin oder Uridin und/oder deren Nukleotide zur Herstellung einer pharmazeutischen Zusammensetzung oder einer Zusammensetzung für die Nährungsergänzung verwendet, welche neben den aktiven Bestandteilen Phosphatidylserin, Cholin, Cytidin oder Uridin und/oder deren Nukleotide des weiteren noch mindestens einen physiologisch verträglichen Träger, Zusatzstoff, Hilfsstoff und/oder Lösungsmittel enthalten.

Derartige Zusammensetzungen enthalten vorzugsweise 1 - 900 mg, bevorzugt 10 - 900 mg, weiter bevorzugt 10 - 700 mg, weiter bevorzugt 50 - 600 mg und insbesondere bevorzugt 100 - 400 mg Phosphatidylserin pro 1 ml einer flüssigen oder pro 1 g einer festen Zusammensetzung.

Ferner enthalten derartige Zusammensetzungen vorzugsweise 10 - 900 mg, bevorzugt 50 - 900 mg, weiter bevorzugt 50 - 600, weiter bevorzugt 100 - 600 mg, noch weiter bevorzugt 150 - 550 mg und insbesondere bevorzugt 200 - 450 mg Pyrimidinnukleosid und/oder Pyrimidinnukleotid pro 1 ml einer flüssigen oder pro 1 g einer festen Zusammensetzung. Sind in der Zusammensetzung mehr als ein Pyrimidinnukleosid oder mehr als ein Pyrimidinnukleotid oder mindestens ein Pyrimidinnukleosid mit mindestens einem Pyrimidinnukleotid vorhanden, so bezieht sich die Mengenangabe auf die Gesamtmenge der Pyrimidinnukleoside bzw. Pyrimidinnukleotide. Sind Kationen wie beispielsweise Natrium vorhanden, so ist deren Masse nicht in der obigen Gewichtsangabe berücksichtigt. Die obigen Gewichtsangaben beziehen sich insofern auf die freie Säureform der Pyrimidinnukleoside und Pyrimidinnukleotide.

Zudem enthalten derartige Zusammensetzungen vorzugsweise 10 - 900 mg, bevorzugt 50 - 900 mg, weiter bevorzugt 50 - 800 mg, weiter bevorzugt 100 - 700 mg, noch weiter bevorzugt 200 - 600 mg und insbesondere bevorzugt 300 - 600 mg Cholin pro 1 ml einer flüssigen oder pro 1 g einer festen Zusammensetzung, wobei die angegebenen Mengenangaben nicht das entsprechende Anion (Chlorid, Citrat usw.) mit umfassen.

Eine Kombination von Phosphatidylserin mit Cytidindiphosphocholin z.B. 1 - 900 mg, 10 - 900 mg, 10 - 700 mg, 50 - 600 mg oder 100 - 400 mg Phosphatidylserin pro 1 ml einer flüssigen oder pro 1 g einer festen Zusammensetzung und 1 - 900 mg, 50 - 900 mg, 100 - 800 mg, 200 - 700 mg oder 300 - 600 mg Cytidindiphosphocholin pro 1 ml einer flüssigen oder pro 1 g einer festen Zusammensetzung enthalten.

Die erfindungsgemäßen pharmazeutischen Zusammensetzungen als auch die Zusammensetzungen für die Ernährungsergänzung werden mit den üblichen festen oder flüssigen Trägerstoffen oder Verdünnungsmitteln und den üblicherweise verwendeten pharmazeutischen Hilfsstoffen entsprechend der gewünschten Applikationsart mit einer geeigneten Dosierung in bekannter Weise hergestellt. Solche Darreichungsformen sind beispielsweise Tabletten, Filmtabletten, Schichttabletten, Dragees, Kapseln, Mikrokapseln, Mikropellets und Pellets, Pillen, Granulaten, Pulver, Puder, Lösungen, Dispersionen, Suspensionen Suppositorien, Emulsionen, Dispersionen, Zäpfchen, Klysma, Gelen, Salben, Sirup oder Depotformen oder Inhalationslösungen bzw. Inhalationspulver. Zudem umfassen die erfindungsgemäßen pharmazeutischen Zusammensetzungen Formulierungen wie Schichttabletten zur kontrollierten und/oder kontinuierlichen Freisetzung des Wirkstoffs sowie Mikroverkapselungen und liposomale Formulierungen als spezielle Darreichungsform. Bevorzugt sind aber flüssige Darreichungsformen beispielsweise in Form von Lösungen, Emulsionen, liposomalen Formulierungen und Dispersionen. Ferner sind Darreichungsformen in Pulverform zum Einrühren in Speisen (Nachspeisen, Quarks, Joghurts, Eis, Puddings usw.) und Getränke (Wasser, Milch, Säfte usw.) bevorzugt.

Derartige Zubereitungen sind unter anderem für die Inhalation oder die intravenöse, intraperitoneale, intramuskuläre, subkutane, orale, transdermale, topikale, intradermale, intragastrale, rektale, intrakutane, intranasale, intrabuccale, perkutane oder sublinguale Verabreichung geeignet, wobei die orale und die intravenöse Gabe bevorzugt ist.

Entsprechende Tabletten können beispielsweise durch Mischen der erfindungsgemäß einsetzbaren Verbindung und/oder deren Salz mit bekannten Hilfsstoffen, beispielsweise inerten Verdünnungsmitteln wie Dextrose, Zucker, Sorbit, Mannit, Polyvinylpyrrolidon, Sprengmitteln wie Maisstärke oder Alginsäure, Bindemitteln wie Stärke oder Gelatine, Gleitmitteln wie Magnesiumstearat oder Talk und/oder Mitteln zur Erzielung eines Depoteffektes wie Carboxypolymethylen, Carboxymethylcellulose, Celluloseacetatphthalat oder Polyvinylacetat, erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Polyvinylpyrrolidon oder Schellack, Gummiarabicum, Talk, Titandioxid oder Zucker hergestellt werden. Dabei kann auch die Drageehülle aus mehreren Schichten bestehen, wobei die oben bei den Tabletten erwähnten Hilfsstoffe verwendet werden können.

Lösungen oder Suspensionen mit der erfindungsgemäßen Wirkstoffkombination werden vorzugsweise mit Wasser als Lösungsmittel zubereitet. Auch Cosolventien wie beispielsweise Ethanol kann in Mengen bis zu 10 Vol.-% eingesetzt werden. Ferner können zusätzlich geschmacksverbessernde Mittel wie Saccharin, Cyclamat oder Zucker sowie Aromastoffe wie Vanillin oder Orangenextrakt enthalten sein. Die flüssigen Zubereitungen können außerdem Suspendierhilfsstoffe wie Natriumcarboxymethylcellulose oder Konservierungsmittel wie p-Hydroxybenzoate enthalten. Die flüssigen Formulierungen umfassen Lösungen, Suspensionen, Sprays sowie Emulsionen und sind vorzugsweise zur oralen, rektalen und parenteralen Applikation geeignet. Bevorzugt sind Lösungen auf Wasserbasis oder Wasser-Propylenglycol-Basis oder liposomale Formulierungen.

Für liposomale Formulierungen können beispielsweise Stoffe wie Phosphatidylcholin, Phosphatidylglycerol, Dimyristoylphosphatidylglycerol, Cholesterol, Dipalmitoylphosphatidylserin, Dipalmitoylphosphatidylcholin und/oder Dimyristoylphosphatidylcholin verwendet werden.

Die Wirkstoffe enthaltende Kapseln können beispielsweise hergestellt werden, indem man den Wirkstoff mit einem inerten Träger wie Milchzucker oder Sorbit mischt und in Gelatinekapseln einkapselt. Kapseln werden beispielsweise aus Methylcellulose, Polyvinylalkohole oder denaturierte Gelatine oder Stärke hergestellt.

Geeignete Suppositorien lassen sich beispielsweise durch Vermischen mit dafür vorgesehenen Trägermitteln wie Neutralfetten oder Polyethylenglykol bzw. Derivaten davon herstellen. Für die Zubereitung von Suppositorien werden bevorzugt niedrigschmelzende Wachse, Fettsäureester und Glyceride eingesetzt.

Als pharmakologisch verträgliche Träger können beispielsweise Lactose, Stärke, Sorbitol, Sucrose, Cellulose, Magnesiumstearat, Dicalciumphosphate, Calciumsulfate, Talk, Mannitol, Ethylalkohol und dergleichen eingesetzt werden. Pulver als auch Tabletten können zu 5 bis 95% aus einem derartigen Träger bestehen.

Als Bindemittel können zudem Stärke, Gelatine, natürliche Zucker, natürliche als auch synthetische Gummis wie beispielsweise Akaziengummi oder Guar-Gummi, Natriumalginat, Carboxymethyl-Cellulose, Polyethylenglycol und Wachse eingesetzt werden. Als Gleitmittel können Borsäure, Natriumbenzoat, Natriumacetat, Natriumchlorid, und dergleichen dienen.

Ferner können den pharmazeutischen Zusammensetzungen noch Sprengmittel, Farbstoffe, Geschmacksstoffe und/oder Bindemittel zugesetzt werden.

Als Sprengmittel können Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, Natrium-Carboxymethylcellulose, microkristalline Cellulose sowie Alginate, Tonerden und Bentonite verwendet werden. Diese Bestandteile können in Mengen von 2 bis 30 Gew.-% eingesetzt werden.

Als Bindemittel können Zucker, Stärke aus Korn, Reis oder Kartoffeln, natürliche Gummis wie Akaziengummi, Gelatine, Tragacanth, Alginsäure, Natriumalginat, Ammonium-Calcium-Alginat, Methylcellulose, Natrium-Carboxymethylcellulose, Hydroxypropyl-methylcellulose, Polyvinylpyrrolidon sowie anorganische Verbindungen wie Magnesium-Aluminum-Silicate zugesetzt werden. Die Bindemittel können in Mengen von 1 bis 30 Gew.-% zugesetzt werden.

Als Gleitmittel können Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natrioumoleat, Polyethylenglycol und Aminosäuren wie Leucin eingesetzt werden. Derartige Gleitmittel können in Mengen von 0,05 bis 15 Gew.-% verwendet werden.

Erfindungsgemäß wird dieKombination umfassend Phosphatidylserin, Chlolin und mindestens einem Pyrimidinnukleosid (z.B. Cytidin oder Uridin) oder mindestens einem Pyrimidinnukleotid (z.B. dCMP, CMP, CDP, dCDP, CTP, dCTP, UMP, dUMP, UDP, dUDP, UTP, dUTP) sowie die pharmazeutischen Zusammensetzungen als auch die nahrungsmittelergänzenden Zusammensetzungen umfassend die Wirkstoffe Phosphatidylserin, Cholin und mindestens ein Pyrimidinnukleosid oder mindestens ein Pyrimidinnukleotid zur Prophylaxe als auch Behandlung von neurodegenerativen und entzündlichen Erkrankungen und Hirnleistungsstörungen insbesondere nach Schlaganfall eingesetzt.

Es gibt viele verschiedene neurodegenerative und entzündliche Erkrankungen, von denen wohl die Alzheimer Erkrankung, Senil-Demenz, ADHS (Attention deficite hyperactivity syndrome), Parkinson, die Huntington Erkrankung, Inflammation Bowel diseases (IBD, wie Colitis ulcerosa und Morbus Crohn) als auch die amyotrophe laterale Sklerose die bekanntesten sind.

Weitere Beispiele für neurodegenerative und entzündliche Erkrankungen, wo die erfindungsgemäße Kombination als auch die erfindungsgemäßen pharmazeutischen Zusammensetzungen wirkungsvoll eingesetzt werden können, sind Hemiatrophie-Hemiparkinson, Parkinsonsyndrom, Demenz, Demenz bedingt durch AIDS, Retinitis Pigmentosa, muskuläre Atrophie, muskuläre Atrophie der Wirbelsäule, paraneoplastische zerebellare Degeneration (PCD), zerebellare Atrophie, extrapyramidale Atrophie, Ataxie, Multiple Sklerose, Phakomatosen, FXTAS (fragile X-associated tremor/ataxia syndrome) auch bezeichnet als das fragile X-Syndrom, progressive supranukleäre Lähmung (PSP: progressive supranuclear palsy), striatonigrale Degeneration (SND), olivoponto-zerebellare Degeneration (OPCD), Shy Drager syndrome (SDS), kortiko-basale Degeneration, Lewy-Körperchen-Demenz, Lewy-Körperchen Krankheit, striato-nigrale Degeneration (SND), idiopathische orthostatische Hypotension (IOH), Multisystematrophien frontotemporale Demenz, Lytico-Bodig Krankheit (Parkinsonismus-Demenz-amyotrophe Lateralsklerose), progressive pallidale Atrophie, Hallervorden-Spatz Erkrankung, x-Chromosom verknüpfte Dystonie (Morbus Lubag), mitochondriale Zytopathie mit striataler Nekrose, Neuroakanthocytose, Restless Leg Syndrom, Polyneuropathien, Wilson'sche Krankheit, Morbus Crohn, Colitis ulcerosa, Irritable Bowel Syndrome als auch multiple System-Atrophie (MSA)..

### Beispiele

Beispiele 3 und 5 fallen nicht unter die Erfindung, wie sie in den Patentansprüchen gekennzeichnet ist.

### Beispiel 1

| 1 Kapsel enthält: | |
|---|---|
| Cholin Citrat | 147 mg |
| Cytidinmonophosphat (Na-Salz) | 161 mg |
| Phosphatidylserin | 50 mg |
| Hilfsstoffe Stärke, Magnesiumstearat ad | 750 mg |

### Beispiel 2

| 1 Kapsel enthält: | |
|---|---|
| Cholin Citrat | 300 mg |
| Uridinmonophosphat (Na-Salz) | 200 mg |
| Phosphatidylserin | 100 mg |
| Hilfsstoffe Methylcellulose, Magnesiumstearat ad | 900 mg |

### Beispiel 3

| 1 Retardtablette enthält: | |
|---|---|
| Cytidindiphosphocholin | 500 mg |
| Phosphatidylserin | 100 mg |
| Hilfsstoffe Hydroxypropylmethylcellulose, Stärke ad | 900 mg |

### Beispiel 4

| 10 ml liposomale Trinklösung enthält: | |
|---|---|
| Cholin Chlorid | 600 mg |
| Cytidin | 550 mg |
| Phosphatidylserin | 300 mg |

Hilfsstoffe: Lecithin, Saccharin, Geschmacksstoff, Wasser.

Phosphatidylcholin wurde zur Herstellung der Liposomen eingesetzt. Es wurde Lipidfilm-Methode verwendet. Die Endkonzentration von Phospholipid beträgt 50 mg/ml.

### Beispiel 5

| 10 ml liposomale Trinklösunq enthält: | |
|---|---|
| Cytidindiphosphocholin | 800 mg |
| Phosphatidylserin | 200 mg |
| Hilfsstoffe: | Phosphatidylcholin, Phosphatidylglycerol, Saccharin, Geschmacks- -stoff, Wasser. |

Phosphatidylcholin und Phophatidylglycerol (Anteil von Phosphatidylglycerol 10 mol%) wurde zur Herstellung der Liposomen eingesetzt. Es wurde Lipidfilm-Methode verwendet. Die Endkonzentration von Phospholipid beträgt 50 mg/ml.

### Beispiel 6

| 30 ml Klysma enthält: | |
|---|---|
| Cholin Bitartrat | 800 mg |
| Uridin (Na-Salz) | 500 mg |
| Phosphatidylserin | 250 mg |
| Natriumbenzoat | 250 mg |
| Xantan Gummi | 100 mg |
| Carbomer | 50 mg |
| Wasser ad | 30000 mg |

### Beispiel 7

Untersuchungen zur Wirkstoffkombination aus Phosphatidylserin, Cytidin und Cholin

Untersucht wurden die möglichen Auswirkungen der Komponenten 1) Phosphatidylserin, 2) Cytidin und Cholin sowie 3) eine Kombination der 3 Komponenten auf die elektrische Hirnaktivität als Indikator für physiologische Veränderungen von Stimmung und kognitiver Hirnleistung.

Mit einem sensitiven EEG-Verfahren wurden bei gesunden Testpersonen die Auswirkungen nach einmaliger oraler Einnahme 1) von Phosphatidylserin, 2) von Cytidin (Na-Salz) und Cholin (Citrat) und 3) von der Kombination der 3 Substanzen untersucht.

Die Untersuchungen wurden an 5 freiwilligen gesunden und nach dem Zufallsprinzip ausgesuchten Testpersonen durchgeführt. Die EEG-Aufnahmen wurden nach 1, 2, 3 und 4 Stunden nach Applikation unter den Bedingungen von 10 Minuten lang geöffnetem Auge und 5 Minuten lang geschlossenem Auge an drei Tagen, welche mindestens eine Woche auseinander lagen, durchgeführt.

Die erste Zusammensetzung bestand nur aus Phosphatidylserin und induzierte eine Abnahme des delta Power (delta Aktivität), begleitet mit leichter aber nicht signifikanter Zunahme des alpha Power (alpha Aktivität).

Die zweite pharmazeutische Zusammensetzung bestehend aus Cytidin und Cholin zeigte keine merkliche Beeinflussung des Frequenzmusters.

Die dritte pharmazeutische Zusammensetzung bestand aus einer Formulierung enthaltend Phosphatidylserin, Cytidin und Cholin. Diese Zusammensetzung war in der Lage, eine stabile Abnahme des delta, alpha2 und beta Wertes (delta, alpha2 und beta Power) zu bewirken. Diese EEG-Änderungen zeigen eine Erhöhung der Hirnaktivität durch diese Zusammensetzung.

### Versuchsbeschreibung Studie 1

5 Testpersonen (2 Frauen und 3 Männer mit einem Durchschnittsalter von 56 Jahren) wurden untersucht. Nach einem Standardfrühstück bestehend aus einem Glas Saft und zwei Scheiben Brot mit Butter und Käse wurde das Basislinien-EEG aufgenommen (10 Minuten unter den Bedingungen des offenen Auges und 5 Minuten mit geschlossenen Augen).

Nach der Gabe der zu untersuchenden Stoffe Phosphatidylserin, Cytidin (Na-Salz) und Cholin (Citrat) erfolgten weitere EEG-Aufnahmen nach 1, 2, 3 und 4 Stunden. Verabreicht wurden eine erste Zusammensetzung, welche 100 mg Phosphatidylserin enthielt, eine zweite Zusammensetzung mit 486 mg Cytidin (Na-Salz) und 590 mg Cholincitrat sowie eine dritte Zusammensetzung, welche sowohl 100 mg Phosphatidylserin als auch 486 mg Cytidin (Na-Salz) und 590 mg Cholincitrat enthielt.

### Durchführung der EEG-Aufnahmen: EEG-Aufnahmen:

Das EEG wurde mittels einer Elektrodenhaube bipolar von 17 Elektroden gemäß dem internationalen 10/20 System mit Cz als physikalischer Referenzelektrode aufgenommen.

Das Rohsignal wurde verstärkt, digitalisiert (2048 Hz/12 bit) und mittels optischer Leitung an einen Computer übermittelt. Die automatische Artefakt-Abweisung des CATEEM^{®}-Systems, welches EEG-Veränderungen verursacht durch Augenzwinkern, Schlucken und Atmung während der Aufnahme herausfiltert, wurde visuell kontrolliert und individuell durch die versuchsdurchführende Person angepaßt.

In diesen Fällen wurden die experimentellen Daten erneut offline ausgewertet mit einem erneut justierten Abweisungsmodus. Die Menge an abgewiesenen Daten wurde automatisch bestimmt und als Prozentangabe bezogen auf die gesamte Aufnahmedauer ausgegeben. Während der gesamten Aufnahme wurde die computer-gesteuerte automatische Artefakt-Abweisung kontinuierlich von einem geschulten Wissenschaftler überwacht und angepaßt (Schober und Dimpfel, Int J Clin Pharmacol Ther Toxicol 1992, 30, 428-430).

Die Signale aller 17 Elektrodenpositionen wurden einer schnellen FourierTransformation (FFT) unterzogen, welche auf 4-Sekunden-Datentakt-Zeiträumen basiert (Hanning Fenster). Die Daten wurden von 0,86 bis 35 Hz unter Verwendung der CATEEM^{®}-Software (MediSyst med. res. & diagn. computer systems, D 35440 Linden, Deutschland) mit einer Frequenz von 128 Hz analysiert.

Bei dieser Software werden die erhaltenen Frequenzspektren in sechs Frequenzbänder unterteilt: delta (1.25 - 4.50 Hz), theta (4.75 - 6.75 Hz), alpha1 (7.00 - 9.50 Hz), alpha2 (9.75 - 12.50 Hz), beta1 (12.75 - 18.50 Hz) and beta2 (18.75 - 35.00 Hz). Diese Frequenzanalyse basiert auf absoluten Spektral-Powerwerten. Die elektrische Aktivität wurde durch Mittelung der Daten einer jeden der 5 Perioden (Referenz und 4 Perioden nach Einnahme der Wirkstoffe) analysiert und als Mittelwerte angegeben.

Für die statistische Auswertung wurde der nicht-parametrische Wilcoxon Test verwendet, um die Daten aus dieser experimentellen Studie mit denselben Perioden einer anderen Studie mit identischem Design aber mit einem Placebo-Arm zu vergleichen. Der Wilcoxon Mann-Whitney U-Test - zweiseitig - wurde verwendet um statistische Unterschiede einerseits bezüglich der gewählten Frequenzbänder und andererseits bezüglich der Mittelwerte aller Aufnahmeelektroden (sogenannte globale Werte) zu detektieren.

### Ergebnisse:

### 1. Elektro-Physiologische Veränderungen induziert durch Phosphatidylserin

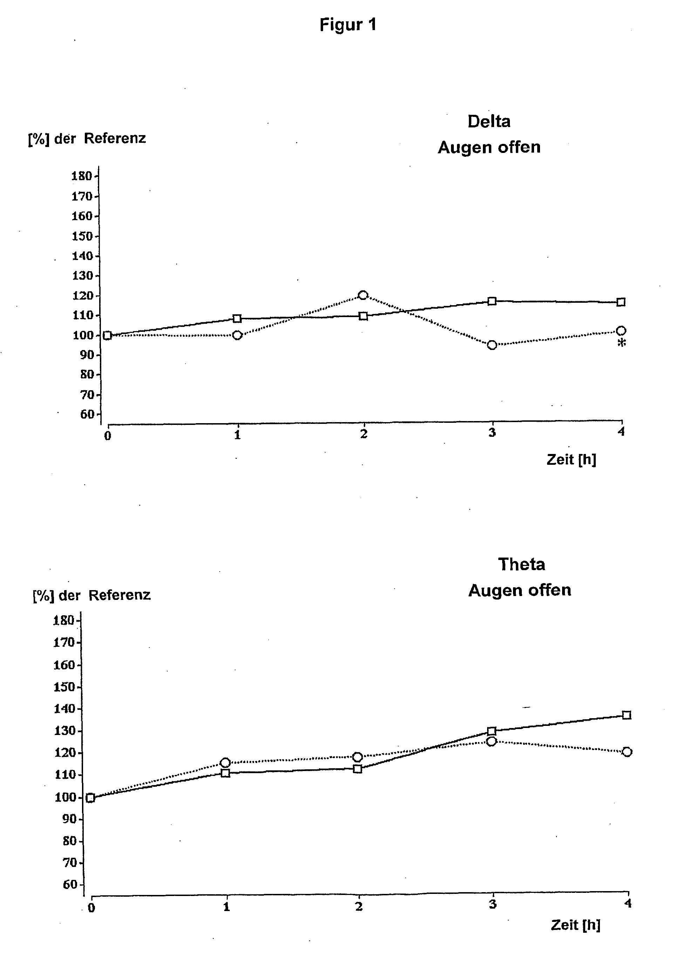

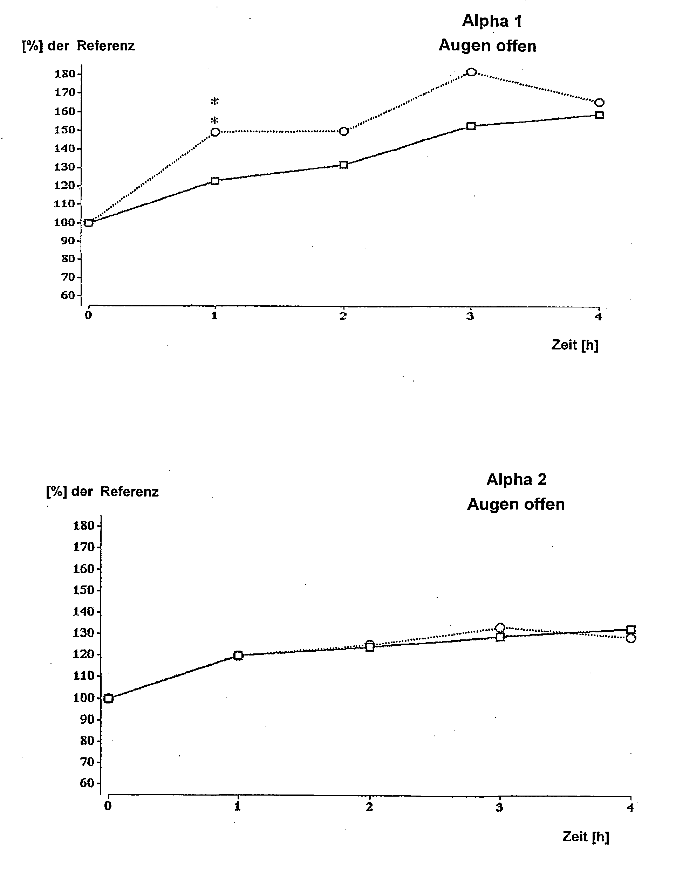

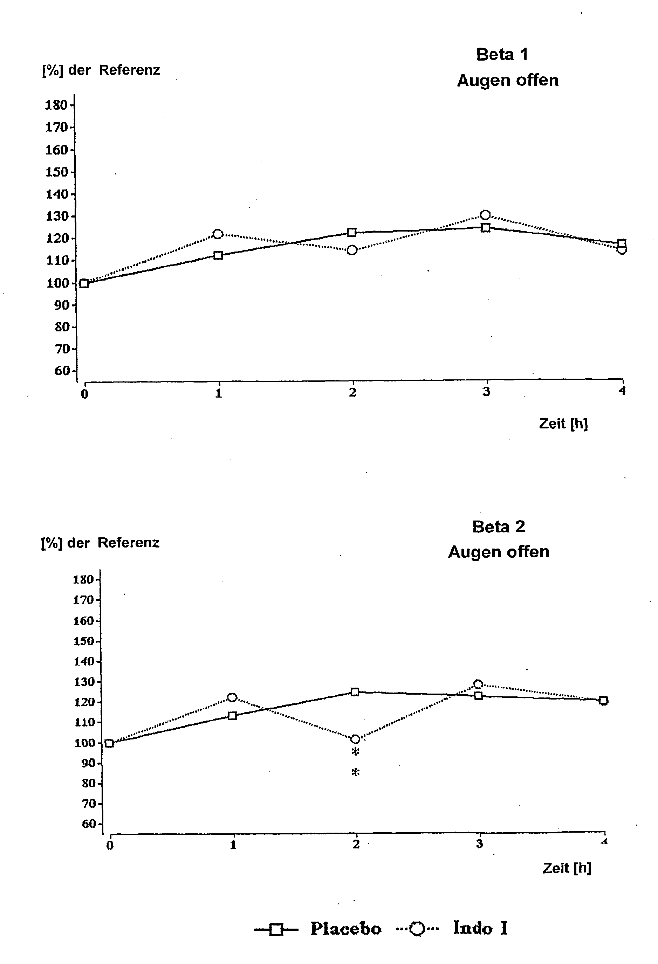

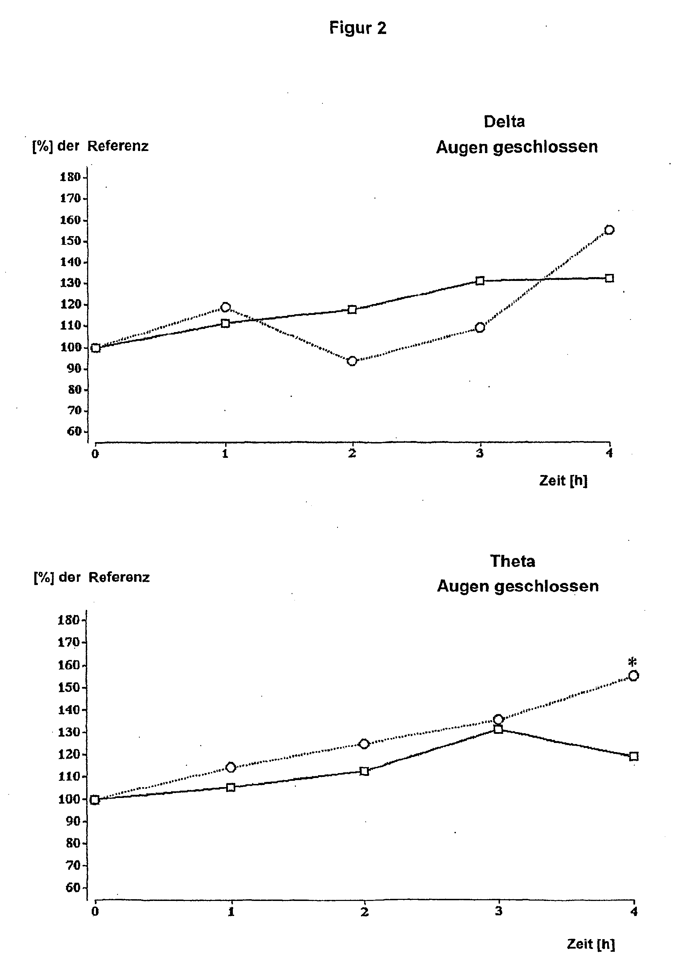

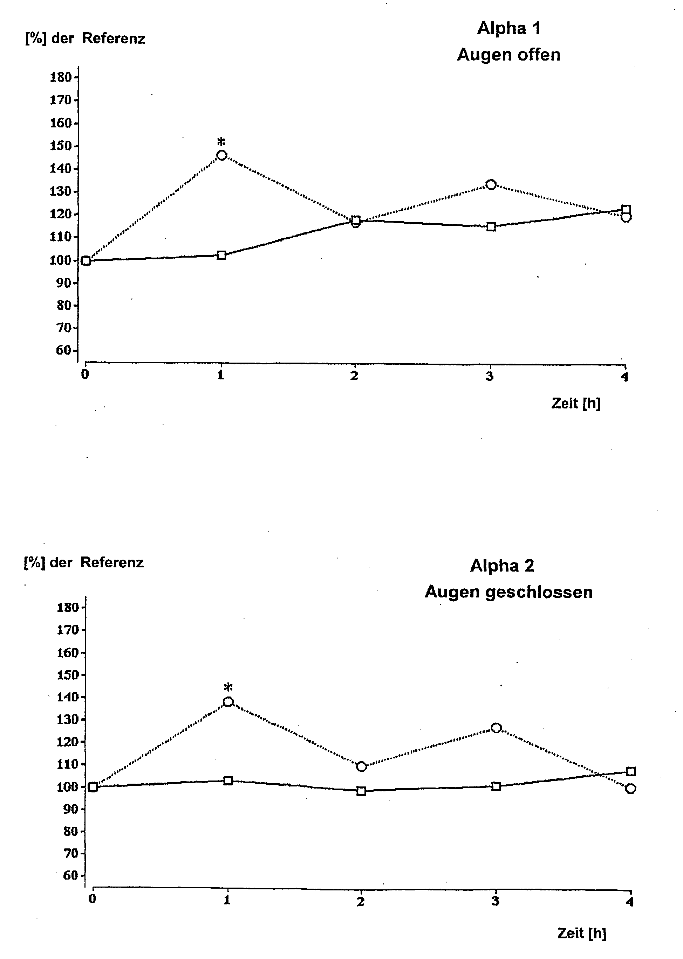

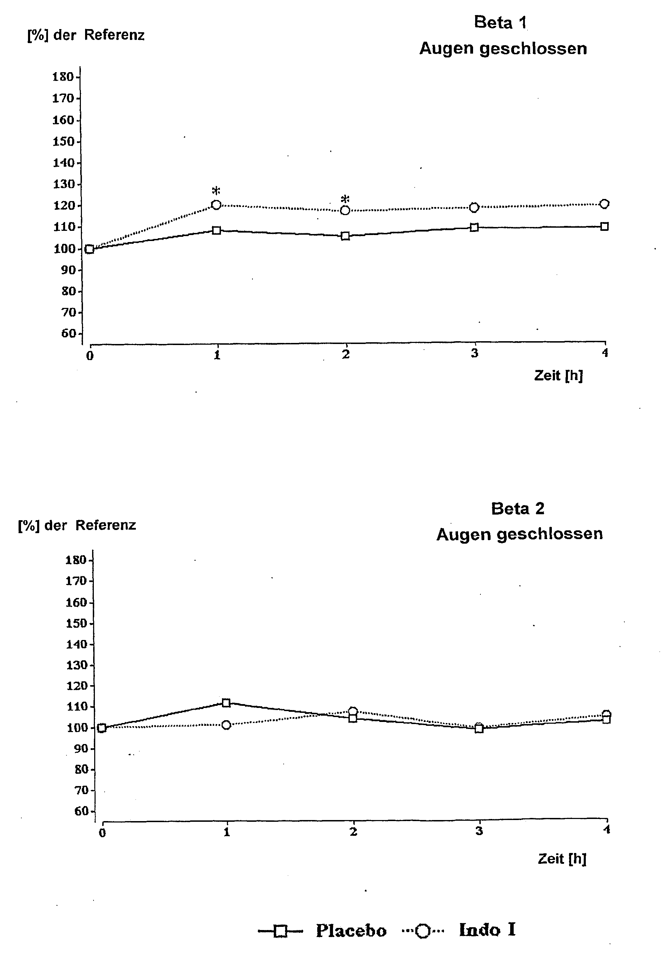

Die erste Zusammensetzung (INDO I) bestand aus Phosphatidylserin und induzierte eine Abnahme der elektrischen delta Aktivität (delta Power), die jedoch nur 4 Stunden nach Applikation statistisch signifikant war (Fig. 1 und 2).

Bei der Abnahme der Beta-Aktivitäten konnte ebenfalls nur bei einem Messzeitpunkt nämlich 3 Stunden nach Verabreichung des Wirkstoffs eine statistisch signifikante Abnahme beobachtet werden (s. Fig. 1 und 2). Unter den Aufnahmebedingungen "Augen offen" wurden die delta Aktivitäten während der zweiten und dritten Stunde vermindert, aber keine statistische Signifikanz konnte nachgewiesen werden (Fig.1 und 2).

### 2. Elektro-Physiologische Veränderungen induziert durch Cytidin plus Cholin

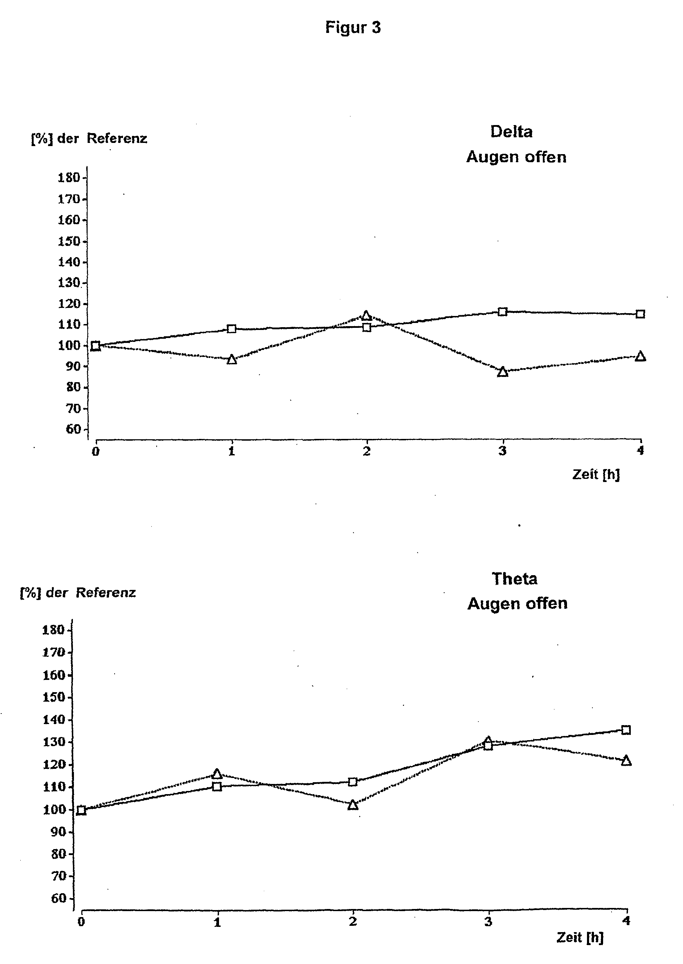

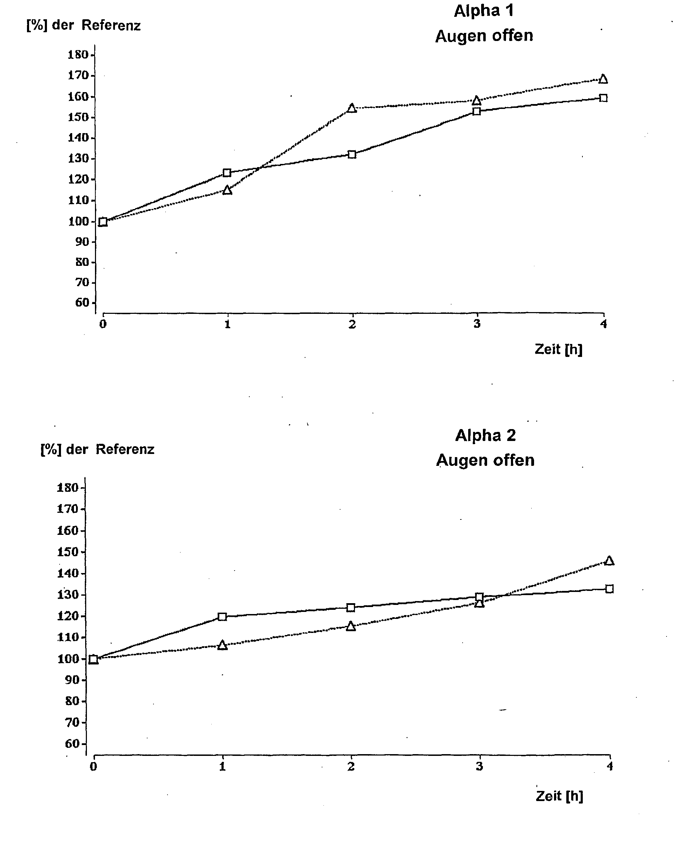

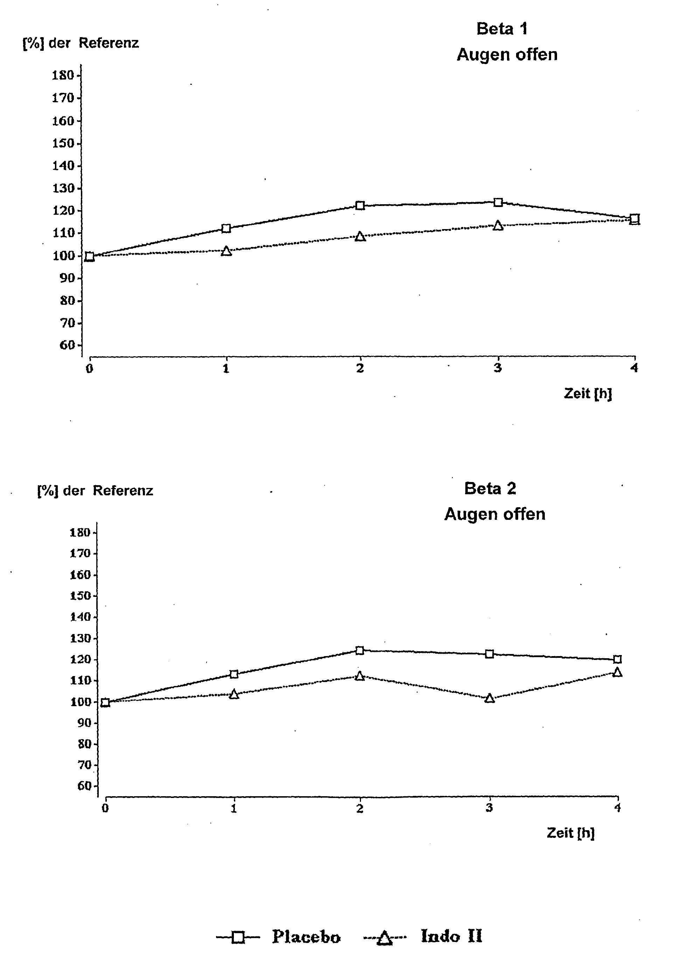

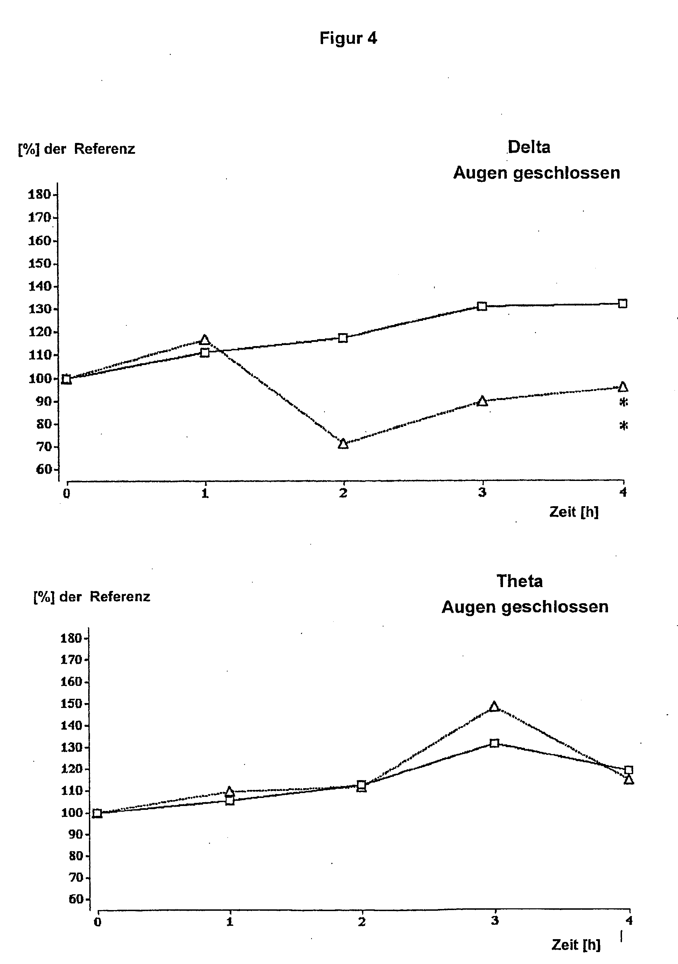

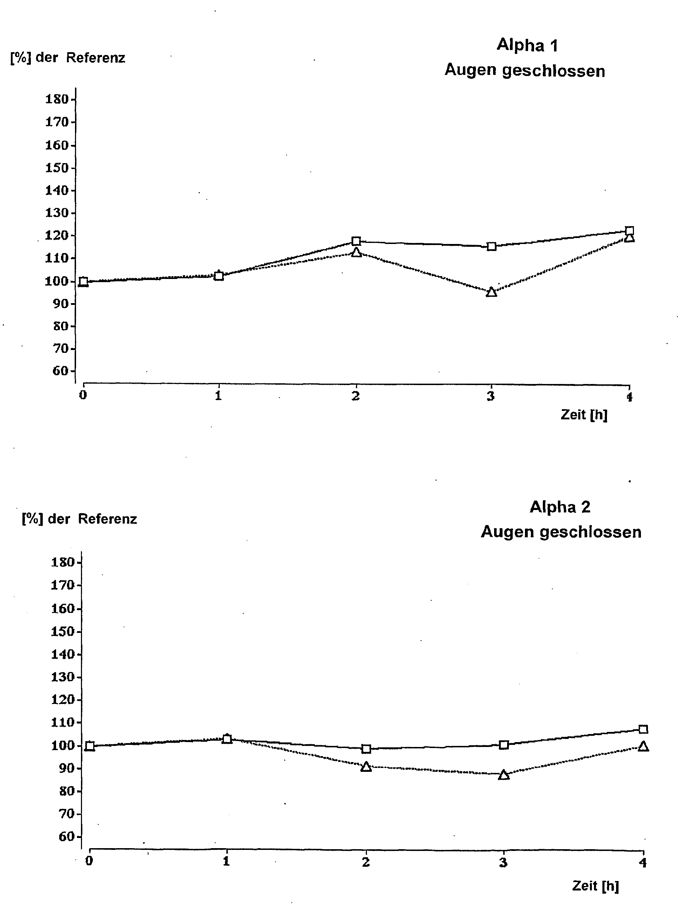

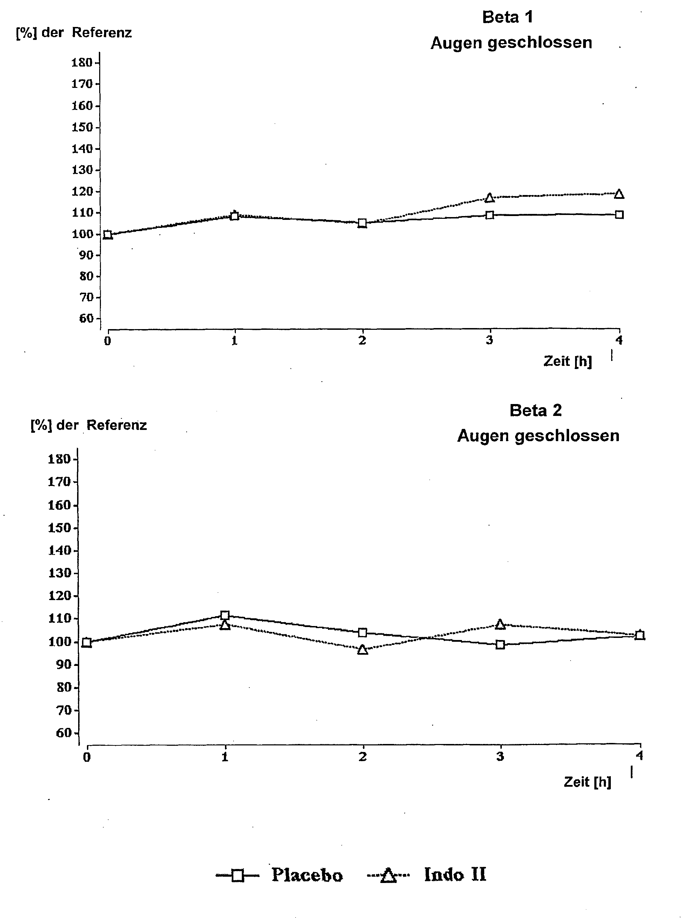

Die zweite Zusammensetzung (INDO II) besteht aus einer Kombination von Cytidin und Cholin und zeigte kaum elektrophysiologische Veränderungen unter "Augenoffen-Bedingungen"(s. Fig. 3). Unter den Aufnahmebedingungen "Augen geschlossen" konnte eine signifikante Abnahme der Delta-Aktivität beobachtet werden, die nur bei einem Messzeitpunkt, nämlich 4 Stunden nach Einnahme der Präparation beobachtet werden konnte (Fig. 4).

### 3. Elektro-Physiologische Veränderungen induziert durch die Kombination aus Phosphatidylserin, Cytidin und Cholin

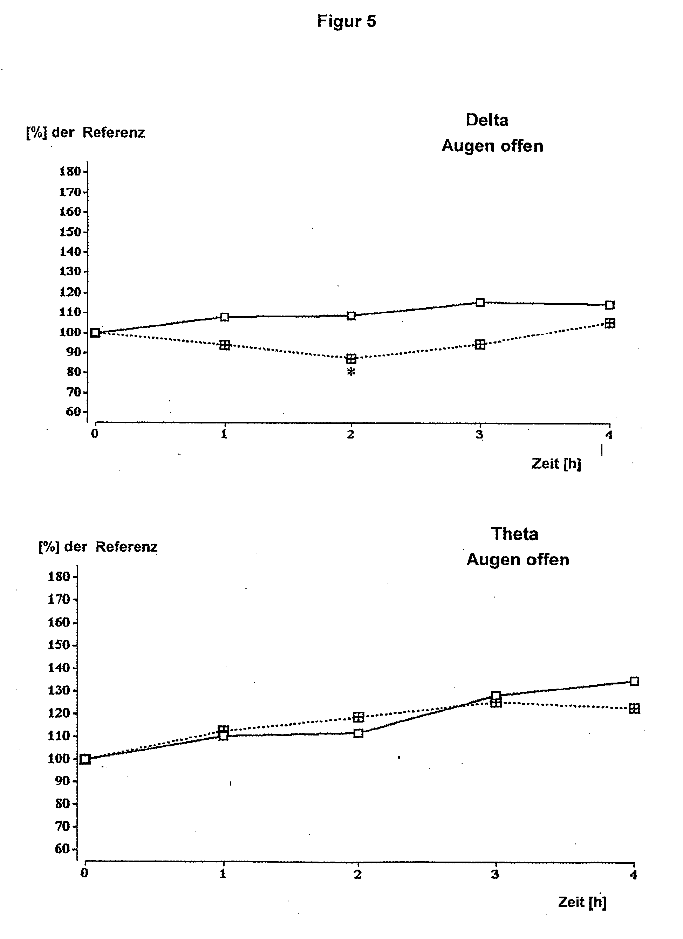

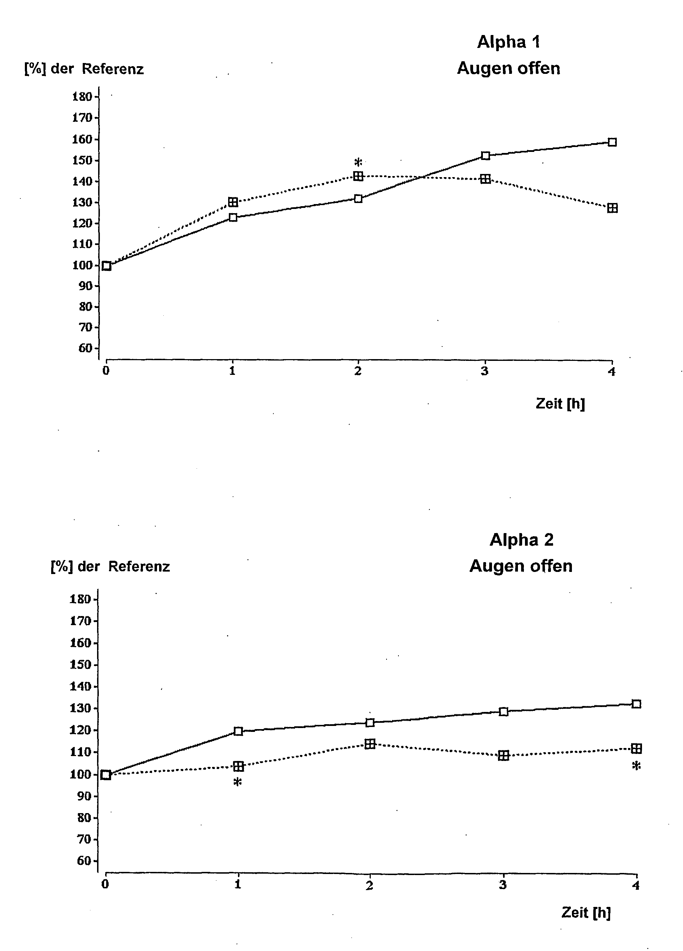

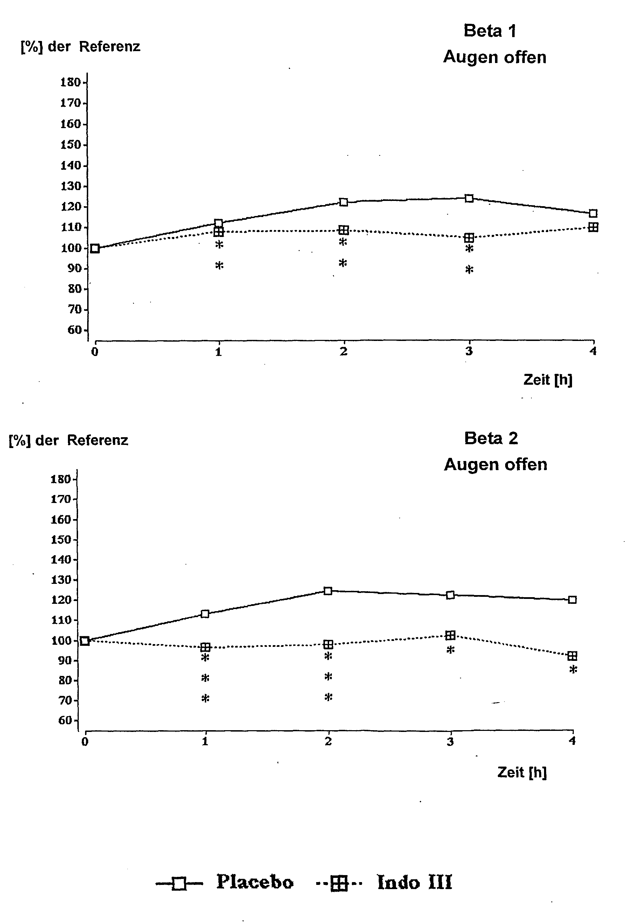

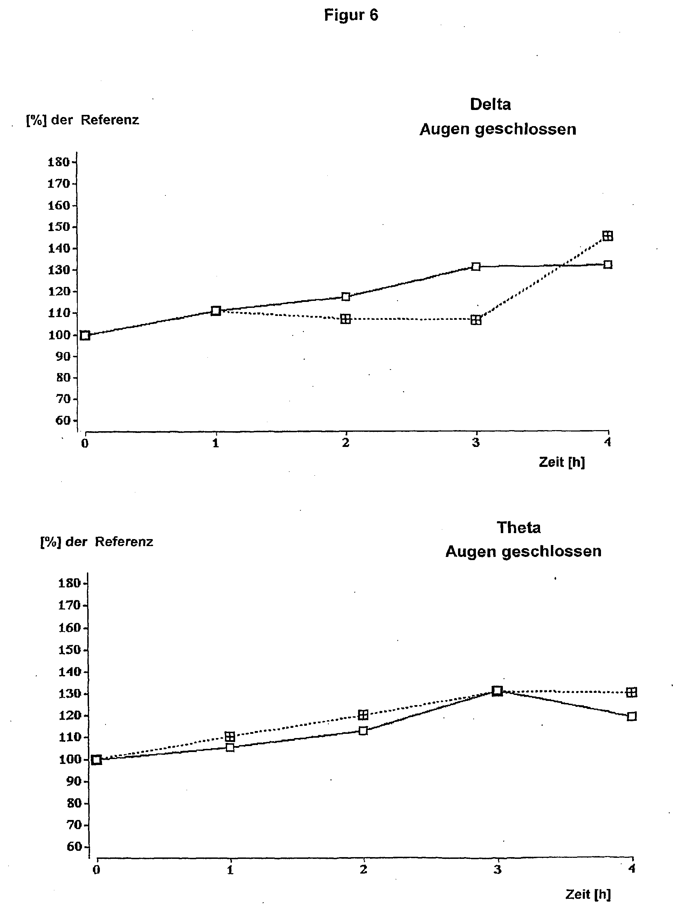

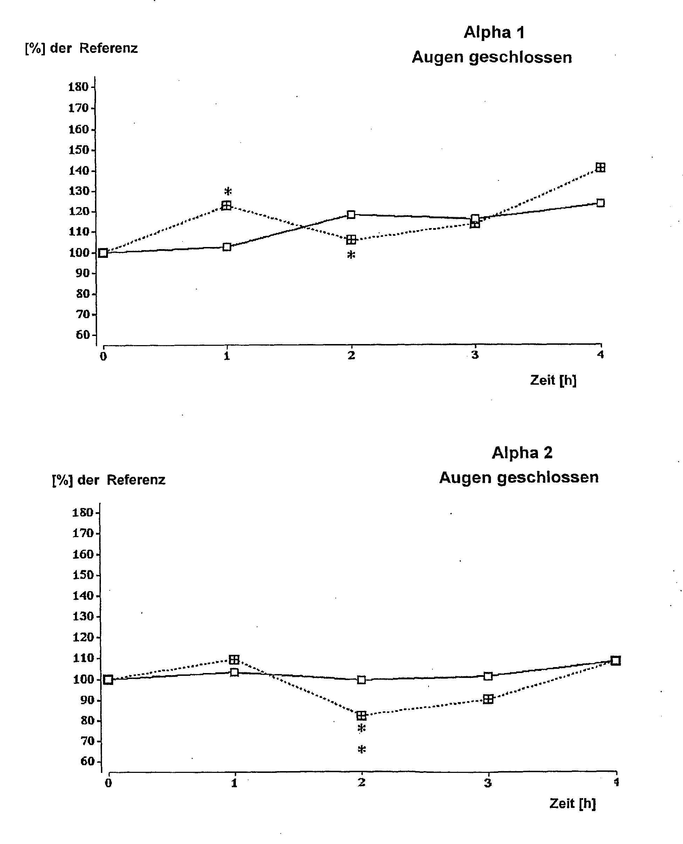

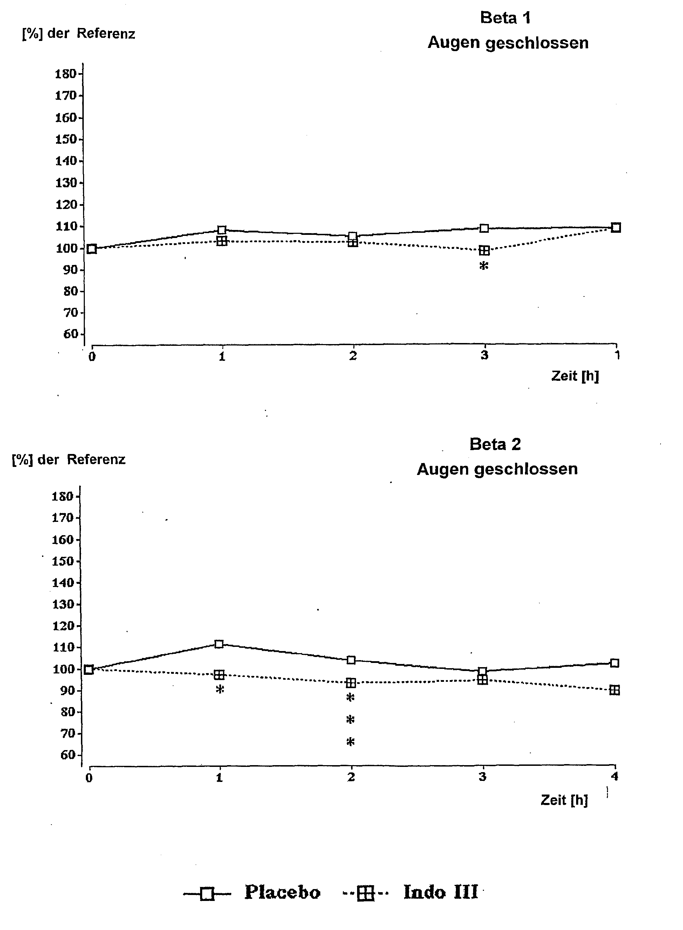

Die dritte Zusammensetzung (INDO III) enthielt Phosphatidylserin, Cytidin und Cholin und bewirkte eine signifikante Abnahme insbesondere der Delta, und Beta-Aktivitäten. Die deutlichsten Effekte wurden beim Beta-Power beobachtet, welche einen statistisch signifikanten Wert unterhalb von 5% Fehlerwahrscheinlichkeit erreichten (s. Fig. 5). Diese Änderungen waren während der gesamten Aufnahmedauer vorhanden (s. Fig. 6).

### Resultate:

Die Gabe von Phosphatidylserin oder insbesondere die Gabe von Cytidindiphosphocholin (= CDP-Cholin, als Vorstufe von Cholin und Cytidin) findet oft Anwendung zur Steigerung der Hirnleistung bei dementen Patienten.

Die Resultate der EEG-Analyse zeigen überraschenderweise die eindeutige Überlegenheit der dreier Kombination von Phosphatidylserin, Cholin und Cytidin gegenüber Phosphatidylserin alleine oder gegenüber der Kombination von Cholin und Cytidin bezüglich der hirnaktivitätssteigernden Wirkung. Die Kombination der 3 Substanzen reduziert signifikant die Delta und Beta-Aktivitäten. Ähnliche Effekte wurden auch für hirnleistungs- und aufmerksamkeitssteigernde, sowie die Wahrnehmung anregende Wirkstoffe beschrieben.

Eine solche signifikante Stimulierung konnte hingegen bei der Applikation der Einzelverbindung Phosphatidylserin als auch bei Gabe der Kombination aus Cytidin und Cholin nicht beobachtet werden.

### Figurenbeschreibung

- Fig. 1: zeigt den zeitlichen Verlauf der EEG Frequenzänderungen in % bezogen auf die Basislinie vor der Einnahme der Zusammensetzung INDO I (nur Phosphatidylserin) für die Aufnahmebedingungen "Augen offen". Die statistische Signifikanz ist durch Sterne gekennzeichnet: * p<0,2; ** p<0,1; *** p<0,05. Die Placebo-Daten wurden einer früheren identischen Studie entnommen.
- Fig. 2: zeigt den zeitlichen Verlauf der EEG Frequenzänderungen in % bezogen auf die Basislinie vor der Einnahme der Zusammensetzung INDO I (nur Phosphatidylserin) für die Aufnahmebedingungen "Augen geschlossen". Die statistische Signifikanz ist durch Sterne gekennzeichnet: * p<0,2; ** p<0,1; *** p<0,05. Die Placebo-Daten wurden einer früheren identischen Studie entnommen.
- Fig. 3: zeigt den zeitlichen Verlauf der EEG Frequenzänderungen in % bezogen auf die Basislinie vor der Einnahme der Zusammensetzung INDO II (Cytidin und Cholin) für die Aufnahmebedingungen "Augen offen". Die statistische Signifikanz ist durch Sterne gekennzeichnet: * p<0,2; ** p<0,1; *** p<0,05. Die Placebo-Daten wurden einer früheren identischen Studie entnommen.
- Fig. 4: zeigt den zeitlichen Verlauf der EEG Frequenzänderungen in % bezogen auf die Basislinie vor der Einnahme der Zusammensetzung INDO II (Cytidin und Cholin) für die Aufnahmebedingungen "Augen geschlossen". Die statistische Signifikanz ist durch Sterne gekennzeichnet: * p<0,2; ** p<0,1; *** p<0,05. Die Placebo-Daten wurden einer früheren identischen Studie entnommen.
- Fig. 5: zeigt den zeitlichen Verlauf der EEG Frequenzänderungen in % bezogen auf die Basislinie vor der Einnahme der Zusammensetzung INDO III (Phosphatidylserin und Cytidin und Cholin) für die Aufnahmebedingungen "Augen offen". Die statistische Signifikanz ist durch Sterne gekennzeichnet: * p<0,2; ** p<0,1; *** p<0,05. Die Placebo-Daten wurden einer früheren identischen Studie entnommen.
- Fig. 6: zeigt den zeitlichen Verlauf der EEG Frequenzänderungen in % bezogen auf die Basislinie vor der Einnahme der Zusammensetzung INDO III (Phosphatidylserin und Cytidin und Cholin) für die Aufnahmebedingungen "Augen geschlossen". Die statistische Signifikanz ist durch Sterne gekennzeichnet: * p<0,2; ** p<0,1; *** p<0,05. Die Placebo-Daten wurden einer früheren identischen Studie entnommen.

### Versuchsbeschreibung Studie 2

Der Einfluss einer oralen Einmalgabe der Kombination Cholin (Citrat), Cytidin (Na-Salz) und Phosphatidylserin auf die Hirnaktivität im Vergleich zu Placebo.

Der Einfluss einer oralen Einmalgabe von Kombination aus 3 Substanzen auf die EEG-Frequenz im Vergleich zu Placebo wurde bei gesunden, freiwilligen Personen untersucht.

Der Versuch wurde analog wie oben beschrieben durchgeführt. Nach dem Verzehr eines Frühstücks, bestehend aus einem Glas Saft und zwei Scheiben Brot mit Butter und Käse die EEG Baseline aufgenommen (10 min bei offenen Augen, 5 Minuten bei geschlossenen Augen). Anschließend erhielten die 5 Versuchsteilnehmer (2 Frauen, 3 Männer, Durchschnittsalter 62 Jahre) eine einmalige Dosis von Kombination aus 3 Substanzen (Cholincitrat 590 mg, Cytidin (Na-Salz) 486 mg und Phosphatidylserin 100 mg) oder Placebo. Nach jeweils 1, 2, 3 und 4 Stunden wurden ein weiteres EEG durchgeführt.

### EEG Aufnahme und Auswertung

Das EEG wurde bipolar mit 17 Oberflächenelektroden entsprechend dem internationalen 10/20 System mit Cz als physikalische Referenzelektrode mit dem CATEEM®-System (Computer aided topographical electroencephalometry) aufgenommen.

Die Signale der 17 Elektrodenpositionen wurden zwischen 0,86 bis 35 Hz unter Verwendung der CATEEM^{®}-Software mit einer Probenfrequenz von 128 Hz analysiert. Die Software teilt das resultierende Frequenzspektrum in 6 Frequenzabschnitte: delta (1,25-4,50 Hz), theta (4,75-6,75 Hz), alpha 1 (7,00-9,50 Hz), alpha 2 (9,75-12,50 Hz), beta1 (12,75-18,50 Hz) and beta2 (18,75-35,00 Hz). Zur Analyse der elektrischen Aktivität wurde der Durchschnittswert der fünf Messperioden (Referenz plus 4 Perioden nach Wirkstoffeinnahme) ermittelt. Zur statistischen Auswertung wurde der nicht parametrische Wilcoxon Test herangezogen.

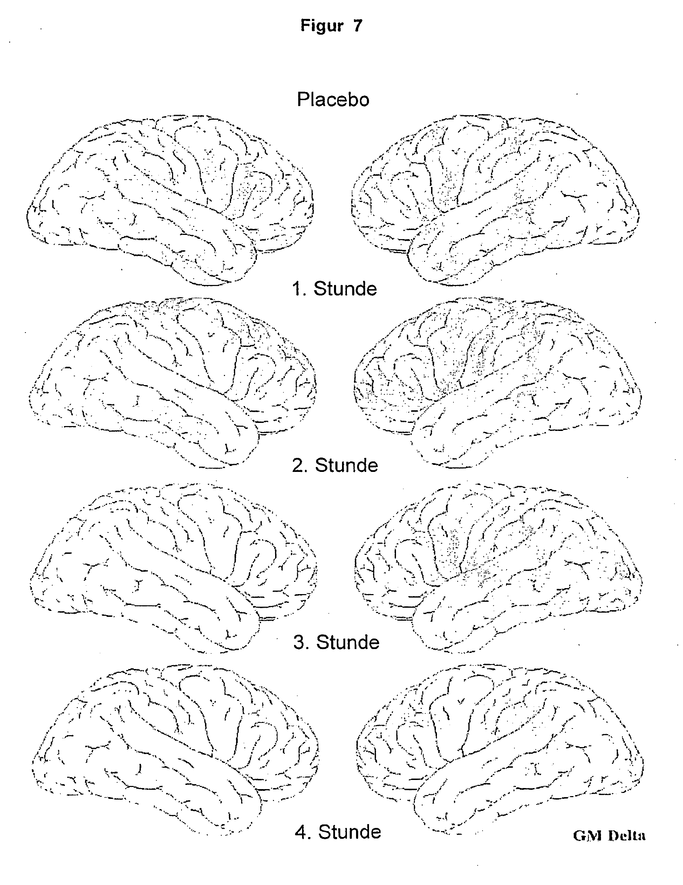

Die Veränderungen der Delta-Frequenzen wurde mit Hilfe der so genannten "glow mode" Methode ausgewertet, visualisiert und farbig dargestellt, wobei die erhöhte Leistung/Aktivität zur helleren Farbgebung und bei niedriger Leistung/Aktivität zu dunkler Farbgebung führt (wie bei glühendem Eisen; s. Fig. 7 - 10).

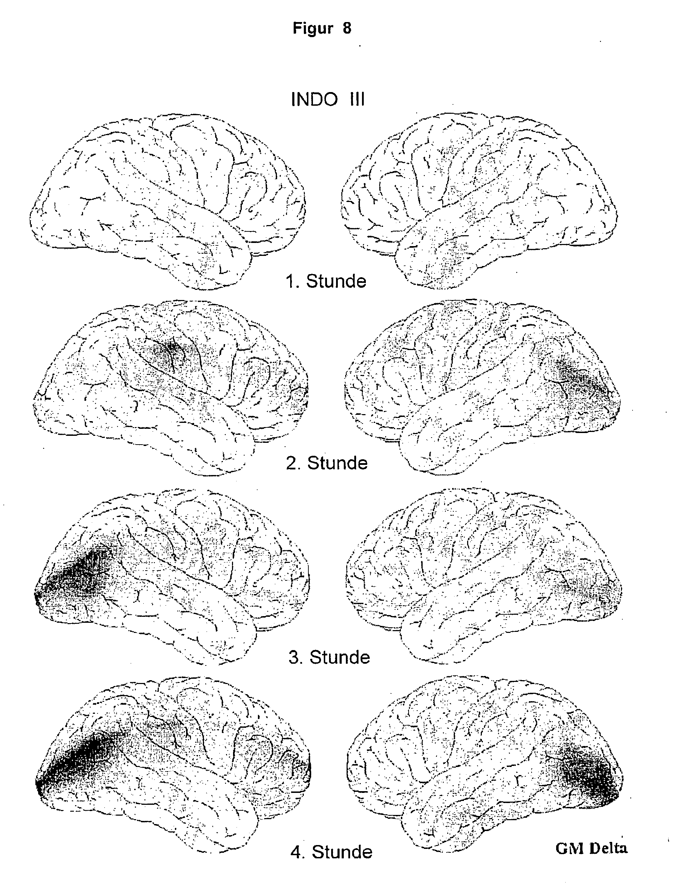

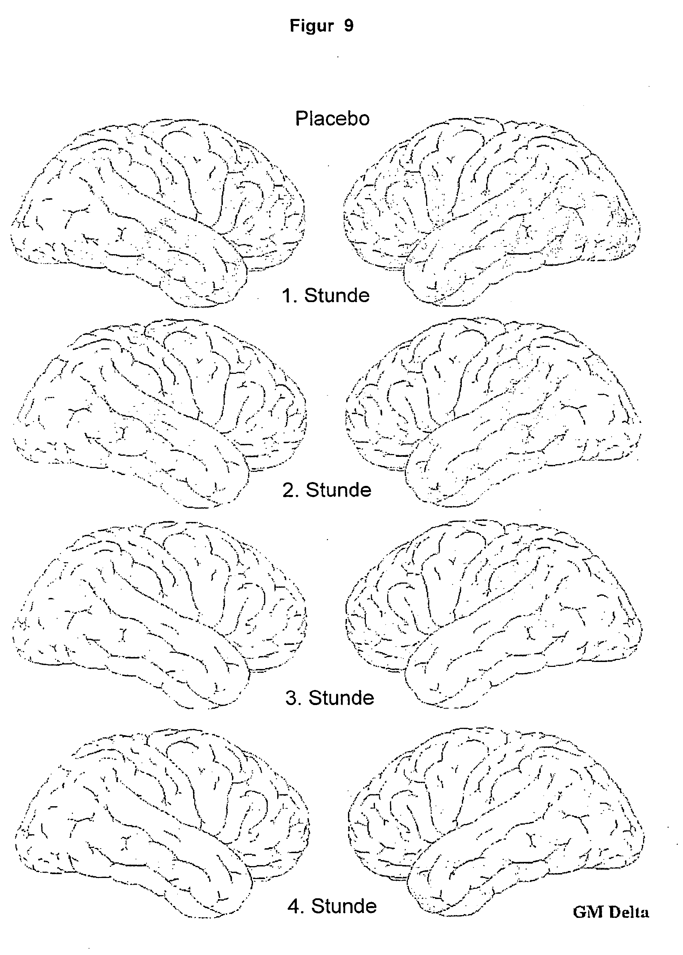

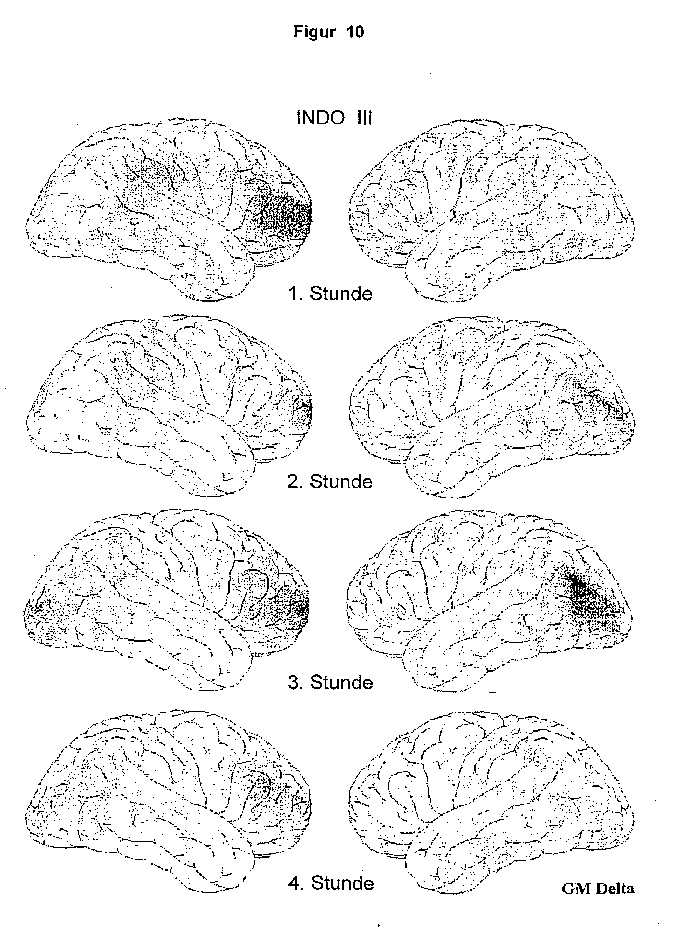

- Fig. 7 - 10: zeigen die visualisiert dargestellten Veränderungen der Delta-Frequenzen, wobei die erhöhte Leistung/Aktivität zur helleren Farbgebung und bei niedriger Leistung/Aktivität zu dunkler Farbgebung führt. Fig. 7 (bei geschlossenen Augen) und Fig. 9 (bei geöffneten Augen) zeigen die visualisierten Resultate bei der Placebo-Gruppe und Fig. 8 (bei geschlossenen Augen) und Fig. 10 (bei geöffneten Augen) zeigen die Testergebnisse mit der Kombination enthaltend Phosphatidylserin, Cytidin und Cholin (INDO III).

### Vergleichbeispiel 8

### Versuchsbeschreibung Studie 3

Wirkung von Cytidindiphosphocholin (CDP-Cholin) und Wirkung von Phosphatidylserin im EEG im Vergleich zur Wirkung der Kombination von Cytidindiphosphocholin und Phosphatidylserin bei oraler Gabe

### Versuchsbeschreibung

4 Testpersonen (2 Frauen und 2 Männer mit einem Durchschnittsalter von 58 Jahren) wurden untersucht. Nach der Einnahme eines Standardfrühstücks bestehend aus einem Glas Saft und zwei Scheiben Brot mit Butter und Käse wurde das Basislinien-EEG aufgenommen (10 Minuten unter den Bedingungen des offenen Auges und 5 Minuten mit geschlossenen Augen).

Nach der oralen Gabe von CDP-Cholin (2 Kapseln enthaltend jeweils 244 mg CDP-Cholin) oder nach der oralen Gabe von Phosphatidylserin (2 Kapseln enthaltend jeweils 250 mg Phosphatidylserin) oder nach der oralen Gabe einer Kombination von Phosphatidylserin und CDP-Cholin (2 Kapseln enthaltend jeweils 244 mg CDP-Cholin und 250 mg Phosphatidylserin) erfolgten weitere EEG-Aufnahmen nach 1, 2 und 4 Stunden.

### Durchführung der EEG-Aufnahmen:

Das EEG wurde mittels einer Elektrodenhaube bipolar von 17 Elektroden gemäß dem internationalen 10/20 System mit Cz als physikalischer Referenzelektrode aufgenommen.

Das Rohsignal wurde verstärkt, digitalisiert (2048 Hz/12 bit) und mittels optischer Leitung an einen Computer übermittelt. Die automatische Artefakt-Abweisung des CATEEM^{®}-Systems, welches EEG-Veränderungen verursacht durch Augenzwinkern, Schlucken und Atmung während der Aufnahme herausfiltert, wurde visuell kontrolliert und individuell durch die versuchsdurchführende Person angepaßt.

In diesen Fällen wurden die experimentellen Daten erneut offline ausgewertet mit einem erneut justierten Abweisungsmodus. Die Menge an abgewiesenen Daten wurde automatisch bestimmt und als Prozentangabe bezogen auf die gesamte Aufnahmedauer ausgegeben. Während der gesamten Aufnahme wurde die computer-gesteuerte automatische Artefakt-Abweisung kontinuierlich von einem geschulten Wissenschaftler überwacht und angepaßt (Schober und Dimpfel, Int J Clin Pharmacol Ther Toxicol 1992, 30, 428-430).

Die Signale aller 17 Elektrodenpositionen wurden einer schnellen FourierTransformation (FFT) unterzogen, welche auf 4-Sekunden-Datentakt-Zeiträumen basiert (Hanning Fenster). Die Daten wurden von 0,86 bis 35 Hz unter Verwendung der CATEEM^{®}-Software (Computer aided topographical electroencephalometry; MediSyst med. res. & diagn. computer systems, D 35440 Linden, Deutschland) mit einer Frequenz von 128 Hz analysiert.

Bei dieser Software werden die erhaltenen Frequenzspektren in sechs Frequenzbänder unterteilt: delta (1.25 - 4.50 Hz), theta (4.75 - 6.75 Hz), alphal (7.00 - 9.50 Hz), alpha2 (9.75 - 12.50 Hz), beta1 (12.75 - 18.50 Hz) and beta2 (18.75 - 35.00 Hz). Diese Frequenzanalyse basiert auf absoluten Spektral-Powerwerten. Die elektrische Aktivität wurde durch Mittelung der Daten einer jeden der 5 Perioden (Referenz und 4 Perioden nach Einnahme der Wirkstoffe) analysiert und als Mittelwerte angegeben.

Für die statistische Auswertung wurde der nicht-parametrische Wilcoxon Test verwendet, um die Daten aus dieser experimentellen Studie mit denselben Perioden einer anderen Studie mit identischem Design aber mit einem Placebo-Arm zu vergleichen. Der Wilcoxon Mann-Whitney U-Test - zweiseitig - wurde verwendet um statistische Unterschiede einerseits bezüglich der gewählten Frequenzbänder und andererseits bezüglich der Mittelwerte aller Aufnahmeelektroden (sogenannte globale Werte) zu detektieren. Insbesondere wurden die delta-Frequenzbänder (1.25 - 4.50 Hz) analysiert.

### Ergbnisse:

1. Veränderungen der delta-Aktivität induziert durch CDP-Cholin unter den Bedingungen "Augen offen"
   Während der 4 Stunden nach der Einnahme von CDP-Cholin (488 mg) konnte eine geringfügige aber im Vergleich zum Placebo-Arm nicht signifikante Veränderung der delta Aktivität beobachtet werden.
   delta Aktivität bei oraler CDP-Cholin-Gabe in Bezug auf die Referenz (Placebo):
   1 h (98%), 2h (102%) 4h (96%)
2. Veränderungen der delta-Aktivität induziert durch Phosphatidylserin unter den Bedingungen "Augen offen"
   Die Verabreichung von insgesamt 500 mg Phosphatidylserin führte nach 4 Stunden zu keinen signifikanten Veränderungen der delta Aktivität im Vergleich zu Placebo;
   delta Aktivität bei oraler Phosphatidylserin-Gabe in Bezug auf die Referenz (Placebo):
   1 h (96%), 2h (94%) 4h (94%)
3. Veränderungen der delta-Aktivität induziert durch die Kombination von Phosphatidylserin und CDP-Cholin unter den Bedingungen "Augen offen" Die Verabreichung von insgesamt 500 mg Phosphatidylserine zusammen mit insgesamt 488 mg CDP-Cholin führte nach 4 Stunden zu einer signifikanten Reduzierung der delta Aktivität im Vergleich zu Placebo;
   delta Aktivität bei oraler Gabe von Phosphatidylserin und CDP-Cholin in Bezug auf die Referenz (Placebo):
   1 h (94%), 2h (89% signifikant) 4h (90% signifikant)

### Zusammenfassung:

Die EEG-Messung der elektrischen Hirnaktivität ist ein anerkanntes Verfahren zur Bestimmung der Wirksamkeit einer Verbindung hinsichtlich Wahrnehmung und Gehirnleistung bzw. Leistungsvermögen des Gehirns.

In diesem Zusammenhang kann die delta Aktivität (delta power) als Kenngröße für die gehirnstimulierende Wirkung eines die Wahrnehmung erhöhenden Wirkstoffs (sogenannter "nootropischer" oder "neurotropischer" Wirkstoff) dienen.

Es wurde beschrieben, dass CDP-Cholin die Gehirnaktivität steigert und die Gemütsstimmung von Patienten mit neurologischen Krankheiten wie beispielsweise Parkinson und senile Demenz verbessert. Ferner war bekannt, dass Phosphatidylserin hilfreich bei der Behandlung von Hirnleistungsstörungen insbesondere Hirnleistungsstörungen nach Schlaganfall und anderen neurologischen Erkrankungen wie z.B. Beeinträchtigung der Gehirnfunktion ist.

Die vorliegenden Resultate zeigen nun, dass die Wirkstoffe CDP-Cholin und Phosphatidylserin alleine appliziert, die Gehirnleistung nicht bzw. nur leicht jedoch nicht signifikant steigern, wohingegen die Kombination aus CDP-Cholin und Phosphatidylserin zu einer signifikanten Reduzierung der delta Aktivität und zu einer signifikanten Steigerung der Hirnleistung führt, wodurch sich neurodegenerative Erkrankungen wie Parkinson, Alzheimer, Demenz, ADHS, Huntington'sche Krankheit und Schlaganfall behandeln lassen bzw. diese Substanzen vorbeugend gegen neurodegenerative Erkrankungen wirken können.

### Beispiel 9

### Versuchsbeschreibung Studie 4

Wirkung der Dreierkombination aus Cytidininonophosphat (CMP) zusammen mit Cholin und Phosphatidylserin (Zusammensetzung I) im EEG im Vergleich zur Wirkung der Dreierkombination aus Uridinmonophosphat (UMP) zusammen mit Cholin und Phosphatidylserin (Zusammensetzung II) bei oraler Gabe

### Versuchsbeschreibung

4 Testpersonen (2 Frauen und 2 Männer mit einem Durchschnittsalter von 58 Jahren) wurden untersucht. Nach der Einnahme eines Standardfrühstücks bestehend aus einem Glas Saft und zwei Scheiben Brot mit Butter und Käse wurde das Basislinien-EEG aufgenommen (10 Minuten unter den Bedingungen des offenen Auges und 5 Minuten mit geschlossenen Augen).

Nach der oralen Gabe von Zusammensetzung I (646 mg CMP (Na-Salz) + 590 mg Cholin (Citrat) + 500 mg Phosphatidylserin) oder nach der oralen Gabe von Zusammensetzung II (648 mg UMP + 590 mg Cholin (Citrat) + 500 mg Phosphatidylserin) erfolgten weitere EEG-Aufnahmen nach 1, 2 und 4 Stunden.

Die Durchführung der EEG-Aufnahmen erfolgt wie in Studie 3 beschrieben mittels einer Elektrodenhaube von 17 Elektroden gemäß dem internationalen 10/20 System mit Cz als physikalischer Referenzelektrode.

### Ergebnisse:

1. Veränderungen der delta-Aktivität aufgrund von Zusammensetzung I unter den Bedingungen "Augen offen"
   Während der 4 Stunden nach der Einnahme von Zusammensetzung I konnte eine merkliche Reduktion der delta Aktivität im Vergleich zum Placebo-Arm beobachtet werden; delta Aktivität bei oraler Gabe von Zusammensetzung I in Bezug auf die Referenz (Placebo):
   1 h (103%), 2h (89% signifikant), 4h (91 % signifikant)
2. Veränderungen der delta-Aktivität aufgrund von Zusammensetzung II unter den Bedingungen "Augen offen"
   Während der 4 Stunden nach der Einnahme von Zusammensetzung II konnte eine merkliche Reduktion der delta Aktivität nach 2 Stunden im Vergleich zum Placebo-Arm beobachtet werden;
   delta Aktivität bei oraler Gabe von Zusammensetzung II in Bezug auf die Referenz (Placebo):
   1 h (99%), 2h (90% signifikant), 4h (95%)

### Zusammenfassung:

Auch die phosphorylierten Derivate von Cytidin und Uridin in Kombination mit Cholin und Phosphatidylserin zeigen einen deutlichen Einfluß auf die Gehirnfunktion.

Zusammensetzung I (CMP + Cholin + Phosphatidylserin) zeigt eine deutliche und signifikante Reduktion der delta Aktivität und somit eine signifikante Stimulierung und Aktivierung der Gehirnfunktion.

Bekanntermaßen kann Cytidin unter physiologischen Bedingungen in Uridin als auch die Cytidinnukleotide (CMP, CDP und CTP) in die entsprechenden phosphorylierten Uridin-Derivate umgewandelt werden. In jüngerer Vergangenheit wurde nun erkannt, dass auch die Umwandlung von Uridin in Cytidin bzw. der Uridinnukleotide in die entsprechenden phosphorylierten Cytidin-Derivate unter physiologischen Bedingungen stattfindet (Trovarelli G. et. al. Neurochem. Res. 7 (10), 1199, 1982; Wurtman R.J. et. al. Biochem. Pharmacol. 60, 989, 2000). Somit ist nicht weiter verwunderlich, dass die positiven Ergebnisse, welche mit Zusammensetzungen enthaltend Cytidin oder ein Cytidinnukleotid auch auf Zusammensetzungen enthaltend Uridin oder ein Uridinnukleotid zu übertragen sind.

Wie oben gezeigt, weist auch die Zusammensetzung II nach 2 Stunden eine signifikante Reduktion der delta Aktivität im Vergleich zu Placebo auf.

### Beispiel 10

### Versuchsbeschreibung Studie 5

Wirkung der Dreierkombination aus Deoxyuridintriphosphat (dUTP) zusammen mit Cholin und Phosphatidylserin (Zusammensetzung III) im EEG im Vergleich zur Wirkung der Zweierkombination (2A) aus Deoxyuridintriphosphat (dUTP) zusammen mit Phosphatidylserin und der Zweierkombination (2B) aus Cholin und Phosphatidylserin bei oraler Gabe

### Versuchsbeschreibung

5 Testpersonen (2 Frauen und 3 Männer mit einem Durchschnittsalter von 56 Jahren) wurden untersucht. Nach der Einnahme eines Standardfrühstücks bestehend aus einem Glas Saft und zwei Scheiben Brot mit Butter und Käse wurde das Basislinien-EEG aufgenommen (10 Minuten unter den Bedingungen des offenen Auges und 5 Minuten mit geschlossenen Augen).

Nach der oralen Gabe von Zusammensetzung III (680 mg dUTP (Na-Salz) + 550 mg Cholin (Citrat) + 550 mg Phosphatidylserin) oder nach der oralen Gabe von 2A (680 mg dUTP (Na-Salz) + 550 mg Phosphatidylserin) oder nach der oralen Gabe von 2B (550 mg Cholin (Citrat) + 550 mg Phosphatidylserin) erfolgten weitere EEG-Aufnahmen nach 1, 2 und 4 Stunden.

Die Durchführung der EEG-Aufnahmen erfolgt wie in Studie 3 beschrieben mittels einer Elektrodenhaube von 17 Elektroden gemäß dem internationalen 10/20 System mit Cz als physikalischer Referenzelektrode.

### Ergebnisse:

1. Veränderungen der delta-Aktivität aufgrund von Zusammensetzung III unter den Bedingungen "Augen offen"
   Während der 4 Stunden nach der Einnahme von Zusammensetzung III konnte eine deutliche Reduktion der delta Aktivität im Vergleich zum Placebo-Arm beobachtet werden;
   delta Aktivität bei oraler Gabe von Zusammensetzung III in Bezug auf die Referenz (Placebo):
   1h (101%), 2h (88% signifikant), 4h (89% signifikant)
2. Veränderungen der delta-Aktivität aufgrund von Zusammensetzung 2A unter den Bedingungen "Augen offen"
   Während der 4 Stunden nach der Einnahme von Zusammensetzung 2A konnte eine leichte jedoch nicht signifikante Reduktion der delta Aktivität im Vergleich zum Placebo-Arm beobachtet werden;
   delta Aktivität bei oraler Gabe von Zusammensetzung 2A in Bezug auf die Referenz (Placebo):
   1 h (100%), 2h (95%), 4h (96%)
3. Veränderungen der delta-Aktivität aufgrund von Zusammensetzung 2B unter den Bedingungen "Augen offen"
   Während der 4 Stunden nach der Einnahme von Zusammensetzung 2B konnte eine leichte jedoch nicht signifikante Reduktion der delta Aktivität im Vergleich zum Placebo-Arm beobachtet werden;
   delta Aktivität bei oraler Gabe von Zusammensetzung 2B in Bezug auf die Referenz (Placebo):
   1h (100%), 2h (96%), 4h (96%)

### Zusammenfassung:

Die Dreierkombination aus Deoxyuridintriphosphat (dUTP) und Cholin und Phosphatidylserin zeigt einen deutlichen und signifikanten Einfluß auf die Gehirnfunktion und Gehirnaktivität.

Die Zweierkombinationen aus Cholin und Phosphatidylserin sowie aus dUTP und Phosphatidylserin vermögen die delta Aktivität leicht jedoch nicht signifikant zu senken.

Die Versuche zeigen somit einstimmig eine synergistische Wirkung der erfindungsgemäßen Kombination gegenüber den Einzelsubstanzen auch aus gegenüber den Zweierkombinationen.

## Patentansprüche

1. Kombination zur Verwendung in einer therapeutischen Behandling, umfassend Phosphatidylserin und Cholin und mindestens ein Pyrimidinnukleosid oder mindestens ein Pyrimidinnukleotid.

2. Kombination zur Verwendung nach Anspruch 1, wobei als Pyrimidinnukleosid Cytidin, Uridin, Deoxycytidin und Deoxyuridin sowie Salze davon und als Pyrimidinnukleotid Cytidinmonophoshat, Deoxycytidinmonophoshat, Cytidindiphosphat, Deoxycytidindiphosphat, Cytidintriphosphat, Deoxycytidintriphosphat, Uridinmonophosphat, Deoxyuridinmonophosphat, Uridindiphosphat, Deoxyuridindiphosphat, Uridintriphosphat und Deoxyuridintriphosphat sowie Salze davon bezeichnet werden.

3. Zusammensetzung, enthaltend die Kombination zur Verwendung gemäß Anspruch 1 oder 2, zur Verwendung in einer therapeutischen Behandlung, zusammen mit mindestens einem physiologisch verträglichen Träger, Zusatzstoff, Hilfsstoff und/oder Lösungsmittel.

4. Zusammensetzung zur Verwendung gemäß Anspruch 3, enthaltend 10 - 900 mg Phosphatidylserin pro 1 ml einer flüssigen oder pro 1 g einer festen Zusammensetzung.

5. Zusammensetzung zur Verwendung gemäß Anspruch 3 oder 4, enthaltend 50 - 900 mg Cholin pro 1 ml einer flüssigen oder pro 1 g einer festen Zusammensetzung.

6. Zusammensetzung zur Verwendung gemäß Anspruch 3, 4 oder 5, enthaltend 50 - 900 mg des mindestens einen Pyrimidinnukleosids und/oder des mindestens einen Pyrimidinnukleotids pro 1 ml einer flüssigen oder pro 1 g einer festen Zusammensetzung.

7. Zusammensetzung zur Verwendung gemäß Anspruch 3 oder 4, enthaltend 50 - 900 mg Cytidindiphosphocholin pro ml einer flüssigen oder pro 1 g einer festen Zusammensetzung.

8. Zusammensetzung zur Verwendung gemäß eines der Ansprüche 3 - 7, geeignet zur oralen, parenteralen, rektalen oder dermalen Applikation.

9. Verwendung einer Kombination umfassend
Phosphatidylserin und Cytidindiphosphocholin
oder
Phosphatidylserin und Cholin und mindestens ein Pyrimidinnukleosid oder mindestens ein Pyrimidinnukleotid
oder einer Zusammensetzung enthaltend besagte Kombination zusammen mit mindestens einem physiologisch verträglichen Träger, Zusatzstoff, Hilfsstoff und/oder Lösungsmittel
zur Herstellung einer pharmazeutischen Zusammensetzung zur Prophylaxe und/oder Behandlung von neurodegenerativen und entzündlichen Erkrankungen.

10. Verwendung der Kombination oder der Zusammensetzung gemäß Anspruch 9, wobei als Pyrimidinnukleosid Cytidin, Uridin, Deoxycytidin und Deoxyuridin sowie Salze davon und als Pyrimidinnukleotid Cytidinmonophoshat, Deoxycytidinmonophoshat, Cytidindiphosphat, Deoxycytidindiphosphat, Cytidintriphosphat, Deoxycytidintriphosphat, Uridinmonophosphat, Deoxyuridinmonophosphat, Uridindiphosphat, Deoxyuridindiphosphat, Uridintriphosphat und Deoxyuridintriphosphat sowie Salze davon bezeichnet werden.

11. Verwendung gemäß Anspruch 9, wobei die neurodegenerativen Erkrankungen ausgewählt werden aus der Gruppe umfassend Alzheimer Erkrankung, Parkinson'sche Krankheit, Huntington'sche Krankheit, Hirnleistungsstörungen, Hirnleistungsstörungen nach Schlaganfall, amyotrophe laterale Sklerose, Senil-Demenz, Restless Leg Syndrom, Polyneuropathien und ADHS.

## Claims

1. Combination for the use in therapeutic treatment comprising phosphatidyl serine and choline and at least one pyrimidine nucleoside or at least one pyrimidine nucleotide.

2. Combination for the use according to claim 1, wherein cytidine, uridine, deoxycytidine and deoxyuridine as well as salts thereof are termed pyrimidine nucleoside, and cytidine monophosphate, deoxycytidine monophosphate, cytidine diphosphate, deoxycytidine diphosphate, cytidine triphosphate, deoxycytidine triphosphate, uridine monophosphate, deoxyuridine monophosphate, uridine diphosphate, deoxyuridine diphosphate, uridine triphosphate and deoxyuridine triphosphate as well as salts thereof are termed pyrimidine nucleotide.

3. Composition containing the combination for the use according to claim 1 or 2 for the use in therapeutic treatment, in combination with at least one physiologically acceptable carrier, additive, auxiliary substance and/ or solvent.

4. Composition for the use according to claim 3, containing 10 - 900 mg phosphatidyl serine per 1 ml of a fluid composition or per 1 g of a solid composition.

5. Composition for the use according to claim 3 or 4, containing 50 - 900 mg choline per 1 ml of a fluid composition or per 1 g of a solid composition.

6. Composition for the use according to claim 3, 4 or 5, containing 50 - 900 mg of the at least one pyrimidine nucleoside and / or of the at least one pyrimidine nucleotide per 1 ml of a fluid composition or per 1 g of a solid composition.

7. Composition for the use according to claim 3 or 4, containing 50 - 900 mg cytidine diphosphocholine per 1 ml of a fluid composition or per 1 g of a solid composition.

8. Composition for the use according to any one of claims 3 - 7, suited for oral, parenteral, rectal or dermal application.

9. Use of a combination containing phosphatidyl serine and cytidine diphospo choline
or
phosphatidyl serine and choline and at least one pyrimidine nucleoside or at least one pyrimidine nucleotide
or a composition containing said combination together with at least one physiologically acceptable carrier, additive, auxiliary substance and / or solvent
for the manufacture of a pharmaceutical preparation for prophylaxis and / or treatment of neurodegenerative and inflammatory diseases.

10. Use of the combination or composition according to claim 9, wherein cytidine, uridine, deoxycytidine and deoxyuridine as well as salts thereof are termed pyrimidine nucleoside, and cytidine monophosphate, deoxycytidine monophosphate, cytidine diphosphate, deoxycytidine diphosphate, cytidine triphosphate, deoxycytidine triphosphate, uridine monophosphate, deoxyuridine monophosphate, uridine diphosphate, deoxyuridine diphosphate, uridine triphosphate and deoxyuridine triphosphate as well as salts thereof are termed pyrimidine nucleotide.

11. Use according to claim 9, wherein the neurodegenerative diseases are selected among the group comprising Alzheimer's disease, Parkinson's disease, Huntington's disease, dementia, dementia subsequent to apoplexia, amyotrophic lateral sclerosis, senile dementia, restless legs syndrome, polyneuropathies, and ADHS.

## Revendications

1. Combinaison pour l'utilisation dans le traitement thérapeutique, comprenant phosphatidylsérine et choline et au moins un pyrimidine-nucléoside ou au moins un pyrimidine-nucléotide.

2. Combinaison pour l'utilisation selon la revendication 1 **caractérisée en ce que** cytidine, uridine, désoxycytidine et désoxyuridine ainsi que les sels de ceux-ci sont indiqué comme pyrimidine-nucléoside et cytidine monophosphate, désoxycytidine monophosphate, cytidine diphosphate, désoxycytidine diphosphate, cytidine triphosphate, désoxycytidine triphosphate, uridine monophosphate, désoxyuridine monophosphate, uridine diphosphate, désoxyuridine diphosphate, uridine triphosphate et désoxyuridine triphosphate ainsi que les sels de ceux-ci sont indiqué comme pyrimidine-nucléotide.

3. Préparation comprenant au moins la combinaison pour l'utilisation selon la revendication 1 ou 2 pour l'utilisation dans le traitement thérapeutique avec au moins un vecteur physiologiquement acceptable, un excipient, adjuvant et/ou diluant.

4. Composition pour l'utilisation selon revendication 3 comprenant 10 à 900 mg phosphatidylsérine par 1 ml d'une composition liquide ou par 1 g d'une composition solide.

5. Composition pour l'utilisation selon revendication 3 ou 4 comprenant 50 à 900 mg choline par 1 ml d'une composition liquide ou par 1 g d'une composition solide.

6. Composition pour l'utilisation selon revendication 3, 4 ou 5 comprenant 50 à 900 mg au moins de pyrimidine-nucléoside et/ou au moins de pyrimidine-nucléotide par 1 ml d'une composition liquide ou par 1 g d'une composition solide.

7. Composition pour l'utilisation selon revendication 3 ou 4 comprenant 50 à 900 mg de la cytidine-diphosphocholine par ml de la composition liquide ou par 1 g de la composition solide.

8. Composition pour l'utilisation selon l'une quelconque des revendications 3 à 7, adaptée à une administration orale, parentérale, rectale ou dermique.

9. Utilisation d'une combinaison comprenant
phosphatidylsérine et cytidine-diphosphocholine
ou
phosphatidylsérine et choline et au moins un pyrimidine-nucléoside ou au moins un pyrimidine-nucléotide
ou une préparation comprenant au moins la dénommée combinaison avec au moins un vecteur physiologiquement acceptable, un excipient, adjuvant et/ou diluant
pour la fabrication d'un agent pharmaceutique pour la prophylaxie et/ou traitement des maladies neurodégénératives et des maladies inflammatoires.

10. Utilisation d'une combinaison ou préparation selon la revendication 9, **caractérisée en ce que** cytidine, uridine, désoxycytidine et désoxyuridine ainsi que les sels de ceux-ci sont indiqué comme pyrimidine-nucléoside et cytidine monophosphate, désoxycytidine monophosphate, cytidine diphosphate, désoxycytidine diphosphate, cytidine triphosphate, désoxycytidine triphosphate, uridine monophosphate, désoxyuridine monophosphate, uridine diphosphate, désoxyuridine diphosphate, uridine triphosphate et désoxyuridine triphosphate ainsi que les sels de ceux-ci sont indiqué comme pyrimidine-nucléotide.

11. Utilisation selon la revendication 9, **caractérisée en ce que** les maladies neurodégénératives est choisi parmi le groupe comprenant: maladie d'Alzheimer, maladie de Parkinson, chorée de Huntington, démence, démence avec attaque d'apoplexie, sclérose latérale amyotrophique, démence-sénile, syndrome des jambes sans repos, polyneuropathies, et trouble déficitaire de l'attention/hyperactivité.
